# EUROPEAN PATENT SPECIFICATION

(11) **EP 1 563 814 B1**
(45) Date of publication and mention of the grant of the patent: **13.06.2007**
(21) Application number: 05010084.1
(22) Date of filing: 09.02.2001
(51) Int. Cl.: A61F 5/41, A61F 2/00, A61B 17/12, A61F 2/26

(54) **Mechanical impotence treatment apparatus**
Mechanische Vorrichtung zur Impotenzbehandlung
Appareil mécanique de traitement de l'impuissance

(30) Priority: 10.02.2000 US 181552 P
(43) Date of publication of application: 17.08.2005
(62) Divisional of application: 01902955.2
(73) Proprietor: Potencia Medical AG, 6341 Baar (CH)
(72) Inventor: Forsell, Peter, 6300 ZUG (CH)
(74) Representative: Strandin, Heléne

(56) References cited:
- EP-A- 0 200 286
- WO-A-96/01597
- WO-A-97/41799
- US-A- 4 723 538
- US-A- 4 829 990
- US-A- 4 958 630
- US-A- 4 982 731
- US-A- 5 048 511

## Description

The present invention relates to a male sexual impotence treatment apparatus, comprising an adjustable restriction device implantable in a male patient, who suffers from sexual impotence, for directly engaging a portion of the normal penile tissue or the prolongation thereof of the patient, and an operable adjustment device implantable in the patient for adjusting the restriction device to temporarily contract said portion of the normal penile tissue or the prolongation thereof to restrict the blood flow leaving the penis, when the patient desires to achieve erection.

Male sexual impotence is a widespread problem. Many different solutions to this problem have been tried. A main solution currently practised and disclosed in for instance U.S. Patent Nos. 5437605 and 4841461 is to implant a hydraulic inflatable/contractible silicon prosthesis in the cavities of the corpora cavernosa of the patient's penis. In fluid connection with this prosthesis is a reservoir implanted in the scrotum. By manual pumping action the prosthesis is filled with fluid from the reservoir to effect errect condition or is emptied of fluid, which returns to the reservoir, to effect flaccid condition.

However, there are several more or less severe disadvantages of this main solution. Above all, the penis is more or less damaged by the operation and it is practically impossible to reverse the operation. Another disadvailtage is that rather strong forces act against this implanted prosthesis resulting in a significant risk of the prosthesis being broken. A further disadvantage is that hard fibrosis created around the reservoir over time may cause malfunction of pumping components. Thus, the created fibrosis will sooner or later become a hard fibrotic layer which may make it difficult to pump the reservoir. Yet a further disadvantage is that the use of hydraulic fluid always entails a risk of fluid leaking from the prosthesis. Furthermore, it is a rather complicated task to manually pump the reservoir when erection is desired.

Another solution to achieve erection is to restrict the blood flow leaving the penis. For example, U.S. Patent No. 4829990 discloses two hydraulically operated inflatable cuffs wrapped around the respective crura. Again, a disadvantage of such a solution is that it entails a risk of hydraulic fluid leaking from the cuffs.

Another example of the solution to restrict the penile blood flow is found in U.S. Patent No. 4828544, which discloses an artificial fistula system surgically implanted and providing a primary fistula between the femoral artery and the femoral vein and a secondary fistula for leading blood from the the primary fistula to the penis. An inflatable balloon engages the primary fistula between the secondary fistula and the vein. The balloon is in fluid connection with a manually compressible reservoir implanted in the scrotum. Besides the risk of fluid leaking from the balloon, a further disadvantage of this latter example is that it requires delicate surgery.

A main disadvantage of the solution to restrict the penile blood flow is the fact that the venous blood vessel system is rather complex and it is difficult to restrict the veinplexa.

Yet another solution is to inject a substance in the vein system to achieve erection. However, injections are painful and complicated for the patient.

The prime object of the present invention is to provide a male sexual impotence treatment apparatus in which the risk of liquid leaking from implanted hydraulic components of the apparatus is substantially reduced or completely eliminated.

A further object of the invention is to provide a male sexual impotence treatment apparatus, which does not require surgical implantation of any fistula system.

Another object of the invention is- to provide a male sexual impotence treatment apparatus, which does not require the use of an injection needle.

These objects are obtained by an apparatus as defined in claim 1.

The restriction device preferably is non-inflatable.

Preferably, the adjustment device adjusts the restriction device in a non-invasive manner. Furthermore, the adjustment device may adjust the restriction device in a non-manual manner and/or in a non-magnetic manner, i.e. magnetic forces may not be involved when adjusting the restriction device.

Furthermore, as opposed to prior art impotence treatment devices the adjustment device of the invention preferably is non-manually operated, i.e. not operated by manual forces or manipulated by touching the skin of the patient, such as by manually compressing a fluid containing balloon implanted in the scrotum. Instead the apparatus of the invention may further comprise a powered operation device for operating the adjustment device.

In the various embodiments hereinafter described the restriction device generally forms an at least substantially closed loop. However, the restriction device may take a variety of different shapes, such as the shape of a square, rectangle or ellipse. The substantially closed loop could for example be totally flat, i.e. thin as seen in the radial direction. The shape of restriction device may also be changed during use, by rotation or movements of the restriction device in any direction.

A physical lumen, such as the corpus cavernosum, crura of the penile tissue or the prolongation thereof, is often easier to restrict by contracting at least two opposite or different side walls of the lumen against each other. The expression "penile tissue and the prolongation thereof" should be understood to mean the penile tissue extended inside the human body and following the pathway of the blood flow leaving the penis i.e. one or several exit veins from the penis, corpus cavernosum, crura or the prolongation thereof. Thus, the restriction device may be designed to perform such a contracting effect of the opposite walls also of a exit vein in the penile prolongation.

Alternatively, the restriction device may comprise an adjustable cuff, a clamp or a roller for bending the vein, corpus cavernosum, crura or the prolongation thereof to restrict the blood flow therein. Such a cuff, clamp or roller may also be utilised for squeezing the vein, corpus cavernosum, crura or the prolongation thereof against human material inside the body of the patient or against implanted structures of the apparatus.

Preferably, the restriction device comprises an elongated restriction member and forming means for forming the restriction member into at least a substantially closed loop around said portion of the tissue, wherein the loop defines a restriction opening, whereby the adjustment device adjusts the restriction member in the loop to change the size of the restriction opening.

The restriction device may be implanted in the base of the patient's penis or the prolongation thereof and preferably may engage the corpus cavernosum, crura or the prolongation thereof of the penis. However, there are several alternative positions of the restriction device that give more or less satisfactory restriction of the blood flow leaving the penis. Thus, as a first alternative the restriction member may extend around both corpora cavernosa or crura of the penis as a single unit. As a second alternative the restriction device may comprise two elongated restriction members extending around respective corpora cavernosa or crura of the patient. As a third alternative the elongated restriction member may encircle one of the exit veins from the penis. As a fourth alternative the restriction device may comprise several restriction members extending around respective exit veins from the penis.

The adjustment device may be incorporated in the restriction device as well as be controlled by hydraulic means.

In accordance with a preferred first adjustment principle, the adjustment device mechanically adjusts the longitudinal extension of the elongated restriction member in a loop form.

In a preferred embodiment of the invention utilising the first adjustment principle, the restriction member comprises a main portion and two elongated end portions, and the adjustment device establishes longitudinal relative displacement between the end portions of the restriction member, so that the size of the restriction opening is adjusted. The forming means may comprise any suitable known or conventional device capable of practising the desired function, such as a spring material forming the elongated restriction member into the loop, so that the restriction opening has a predetermined size, and the adjustment device may adjust the restriction member against the spring action of the spring material. In other words, the restriction member may comprise a spring clip. The spring material may be integrated in the restriction member.

Preferably, the adjustment device comprises a movement transferring member, suitably a drive wheel, in engagement with at least one of the end portions of the restriction member and operable to displace the one end portion relative to the other end portion of the restriction member. The drive wheel may advantageously be in engagement with both of the end portions of the restriction member and be operable to displace said end portions relative to each other. An elongated flexible drive shaft may be operatively connected to the drive wheel, for transferring manual or motor generated power from a location remote from the restriction member. In its simplest embodiment, the drive wheel may comprise a pulley in frictional engagement with the restriction member. As an alternative, a gear rack may be formed on at least one of the end portions of the restriction member and the drive wheel may comprise a gear wheel in mesh with the gear rack. Other suitable known or conventional mechanisms may also or alternatively be used as the adjustment device.

The movement transferring member may alternatively comprise at least one cylinder and a piston, which is movable therein and is connected to one of the end portions of the restriction member, the piston being operable to longitudinally displace the one end portion of the restriction member relative to the other end portion of the restriction member. Alternatively, the movement transferring means may comprise two interconnected cylinders and two pistons in the respective cylinders connected to said end portions, respectively, of the restriction member, the pistons being operable to longitudinally displace the end portions of the restriction member relative to each other. Other known or conventional devices also or alternatively can be used as the movement transferring member.

A motor, which is fixed relative to the main portion of the restriction member and has a rotating drive shaft operatively connected to the movement transferring member, may be positioned relative to the elongated restriction member such that the drive shaft extends transverse thereto. Alternatively, the motor may be positioned relative to the elongated restriction member such that the drive shaft extends substantially tangentially to the loop of the restriction member.

In another embodiment of the invention utilizing the first adjustment principle, the elongated restriction member is longitudinally resilient and the adjustment device comprises a contraction device for longitudinally contracting the resilient restriction member. Preferably, the elongated restriction member comprises a substantially non-resilient main portion and an end portion forming an elongated helical spring, which is contractible by the contraction device. The contraction device may suitably comprise an elongated flexible pulling member connected to the main portion of the restriction member and extending through the helical spring to contract the helical spring against an arresting member, which is fixed relative to the main portion of the restriction member. The pulling member may extend in an elongated tube joined at one end thereof to the arresting member, so that a motor remote from the restriction member may be attached to the other end of the elongated tube and pulls the pulling member through the tube to contract the helical spring.

In yet another embodiment of the invention utilizing the first adjustment principle, the elongated restriction member comprises an elongated helical spring having a free end, and a body to which the spring is nonrotatably secured at its opposite end. The adjustment device rotates the helical spring in one direction to enlarge the coils of the helical spring to longitudinally contract the spring and to rotate the spring in the opposite direction to reduce the size of the coils of the spring to longitudinally extend the spring. As a preferred alternative, the restriction member comprises a further elongated helical spring having a free end and nonrotatably secured to the body at its opposite end, and the adjustment device comprises a drive shaft having two opposite end portions connected to the springs, respectively, at their free ends, the helical coils forming left and right hand helices, respectively. The adjustment device may alternatively comprise a gearing having an input shaft and two opposite aligned output shafts connected to the helical springs, respectively, at their free ends, the input shaft being connected to said output shafts so that the output shafts rotate in the opposite directions upon rotation of the input shaft, the helical coils forming the same helices.

In accordance with a second adjustment principle, the adjustment device mechanically adjusts the restriction member so that at least a portion of a radially innermost circumferential confinement surface formed by the restriction member is substantially radially displaced.

In one embodiment of the invention utilizing the second adjustment principle, the restriction member comprises an elongated voltage responsive element forming part of the confinement surface and capable of bending into a bow in response to a voltage applied across the element, the radius of curvature of the bow being adjustable by changing the level of the voltage.

In another embodiment of the invention utilizing the second adjustment principle, the adjustment device changes the diameter of an elastic annular element of the restriction member, which forms the confinement surface. Preferably, the forming means comprises a substantially -rigid outer annular element coaxially surrounding the elastic annular element, and the adjustment device comprises means for pulling the elastic annular element radially outwardly towards the outer annular element to expand the elastic annular element. For example, the pulling means may comprise a plurality of threads secured to the elastic annular element along the circumference thereof and running from the elastic annular element via guide members attached to the outer annular element.

In yet another embodiment of the invention utilizing the second adjustment principle, the forming means comprises a substantially rigid outer annular element, and the restriction member comprises an elongated helical spring extending internally along the outer annular element and contacting the latter. The helical spring forms part of the circumferential confinement surface and has a free end. The restriction member further comprises a body to which the spring is nonrotatably secured at its opposite end. The adjustment device rotates the helical spring in one direction to enlarge the coils of the spring to contract the circumferential confinement surface and rotates the spring in the opposite direction to reduce the size of the coils of the spring to expand the circumferential confinement surface. As an alternative, which is preferred, the restriction member comprises two elongated helical springs forming part of the circumferential confinement surface and connected to the body of the restriction member. The adjustment device rotates each spring in one direction to enlarge the coils of the spring to contract the circumferential confinement surface and rotates the spring in the opposite direction to reduce the size of the coils of the spring to expand the circumferential confinement surface.

In accordance with a third adjustment principle, the restriction member comprises at least two separate elements, at least one of which is pivoted so that it may turn in a plane in which the the restriction member extends, and the adjustment device turns the pivoted element to change the size of the restriction opening. Preferably, the restriction member comprises a plurality of separate pivoted elements disposed in series, each pivoted element being turnable in the plane, and the adjustment device turns all of the pivoted elements to change the size of the restriction opening. For example, the pivoted elements may comprise lamellae arranged like the conventional adjustable aperture mechanism of a camera.

In accordance with a fourth adjustment principle, the adjustment device folds at least two foldable frame elements of the restriction member towards each other. Preferably, the foldable frame elements comprise two substantially or partly semi-circular frame elements which are hinged together so that the semi-circular elements are swingable relative to each other from a fully open state in which they form part of a circle to a fully folded state in which they form part of a semi-circle. The same principal may be used with the swingable parts mounted together in one end and not in the other end. Alternatively, the restriction device may comprises two preferable rigid articulated clamping elements positioned on opposite sides of a vein like the blades of a scissor, and the adjustment device turns the clamping elements toward each other to clamp the vein, corpus cavernosum, crura or the prolongation thereof between the clamping elements, thereby restricting the blood flow in the vein, corpus cavernosum, crura or the prolongation thereof.

In accordance with a fifth adjustment principle, the adjustment device turns the restriction member around a longitudinal extension thereof, the elongated restriction member being elastic and varying in thickness as seen in a cross-section therethrough. Suitably, the elongated restriction member comprises an elastic belt.

In accordance with a sixth adjustment principle, the adjustment device changes the size of the restriction opening such that the outer circumferential confinement surface of the restriction member is changed.

In accordance with a seventh adjustment principle, the adjustment device changes the size of the restriction opening such that the outer circumferential confinement surface of the restriction member is unchanged.

In accordance with an eighth adjustment principle, the elongated restriction member may be flexible, and the adjustment device pulls a first portion of the flexible restriction member from a second portion of the flexible restriction member opposite the first portion in the loop to squeeze a vein, the corpus cavernosum, the crura or the prolongation thereof between two opposite lengths of the elongated flexible restriction member to restrict the blood flow in the vein, corpus cavernosum, crura or the prolongation thereof.

In accordance with a ninth adjustment principle, the restriction device comprises two rigid elements on opposite or different sides of a vein, the corpus cavernosum, the crura or the prolongation thereof, and the adjustment device decreases the distance between the rigid elements to squeeze the vein, corpus cavernosum, crura or the prolongation thereof between the rigid elements, thereby restricting the blood flow in the vein, corpus cavernosum, crura or the prolongation thereof.

In accordance with a tenth adjustment principle, the restriction device bends or rotates a portion of a vein, the corpus cavernosum, the crura or the prolongation thereof to restrict the blood flow in the same. For example, the restriction device may comprise at least two bending members, such as cylindrical or hour-glass shaped rollers, positioned on opposite or different sides of the vein and displaced relative to each other along the vein, corpus cavernosum, crura or the prolongation thereof, and the adjustment device may move the bending members against the vein, corpus cavernosum, crura or the prolongation thereof to bend the latter to restrict the blood flow in the vein, corpus cavernosum, crura or the prolongation thereof. Suitably, the displacement members may comprise rollers. The restriction device may also rotate a portion of the esophagus or stomach. The bending or rotating members may take any shape. With the prolongation of the corpus cavernosum or crura should be understood the penile tissue extended inside the human body and following the pathway of the blood flow leaving the penis.

Alternetively, the two bending members one placed more distal than the other may be rotated in opposite direction relative to each other. With interconnecting material for example flexible bands between the holding members a restriction will occur between the bending members when they are rotated.

The restriction device may in all applicable embodiments take any shape and be either hydralic or non-inflatable.

In all of the above-described embodiments of the invention the adjustment device is conveniently operated by any suitable motor, preferably an electric motor, which may be fixed directly to or be placed in association with the restriction device, or alternatively be located remote from the restriction device, advantageously in the abdomen or subcutaneously. In the latter alternative the motor is advantageously connected to the adjustment device by a flexible power transmission conduit to permit a suitable positioning of the motor in the abdomen of the patient. The motor may be manually activatable, for example by an implanted switch.

According to the invention, however, the adjustment device is operable by an operation device comprising hydraulic means. The hydraulic operation device may advantageously include hydraulic servo means to facilitate manual activation. As an alternative, the hydraulic device may be powered by an electric motor, which may be manually activatable or controlled by remote control means. The components of such a hydraulic operation device may be placed in association with the restriction device and/or be located at a suitable place in the abdomen or subcutaneously.

More specifically, a reservoir is provided containing a predetermined amount of fluid for supplying the hydraulic operation device with fluid. The reservoir defines a chamber for the predetermined amount of fluid and the hydraulic operation device changes the size of the chamber. The hydraulic operation device may comprise first and second wall portions of the reservoir, which are displaceable relative to each other to change the size of the chamber of the reservoir. The first and second wall portions of the reservoir may be designed to be displaceable relative to each other by manual manipulation thereof, preferably to permit manual pushing, pulling or rotation of any of the wall portions in one direction. Alternatively, the wall portions may be displaceable relative to each other by magnetic means (such as a permanent magnet and magnetic material reed switch, or other known or conventional magnetic devices), hydraulic means or electrical control means such as an electric motor. The magnetic means, hydraulic means, or electrical control means may all be activated by manual manipulation, preferably using a subcutaneously located manually manipulatable device. This control may be indirect, for example via a switch.

The hydraulic operation device may operate the adjustment device with fluid from the reservoir in response to a predetermined first displacement of the first wall portion of the reservoir relative to the second wall portion of the reservoir, to adjust the restriction device to release the tissue, and to operate the adjustment device with fluid from the reservoir in response to a predetermined second displacement of the first wall portion of the reservoir relative to the second wall portion of the reservoir, to adjust the restriction device to restrict the blood flow leaving the penis. In this embodiment, no pump is used, only the volume of the reservoir is varied. This is of great advantage compared to the solution described below when a pump is used to pump fluid between the reservoir and the adjustment device because there is no need for a non-return valve and it is still possible to have fluid going both to and from the reservoir.

As an alternative, the hydraulic operation device may comprise a pump for pumping fluid between the reservoir and the adjustment device. The pump may pump fluid both to and away from the adjustment device, or hydraulic means controlling the adjustment device. A mechanical manual solution is not part of the present invention in which it is possible to pump in both directions just by pushing an activating member in one direction. Another alternative is a pump pumping in only one direction and an adjustable valve to change the direction of fluid to either increase or decrease the amount of fluid in the reservoir. This valve may be manipulated manually, mechanically, electrically, magnetically, or hydraulically. Any kind of motor could of course be used for all the different operations as well as wireless remote solutions. The pump may comprise a first activation member for activating the pump to pump fluid from the reservoir to the adjustment device and a second activation member for activating the pump to pump fluid from the adjustment device to the reservoir. The activation members may be operable by manual manipulation, preferably to permit manual pushing, pulling or rotating thereof in one direction. Suitably, at least one of the activation members is adapted to operate when subjected to an external pressure exceeding a predetermined magnitude.

Alternatively, at least one of the first and second activating members may be operable by magnetic means, hydraulic means or electrical control means such as an electric motor. The magnetic means, hydraulic means, or electrical control means may all be activated by manual manipulating means preferably located subcutaneously. This activation may be indirect, for example via a switch.

Advantageously, a servo means could be used. With servo means less force is needed for operating the adjustment device. The term "servo means" encompasses the normal definition of a servo mechanism, *i.e.* an automatic device that controls large amounts of power by means of very small amounts of power, but may alternatively or additionally encompass the definition of a mechanism that transfers a weak force acting on a moving element having a long stroke into a strong force acting on another moving element having a short stroke. The servo means may comprise a motor, preferably an electric motor, which may be reversible.

Alternatively, a reverse servo may be employed. The term "reverse servo" is to be understood as a mechanism that transfers a strong force acting on a moving element having a short stroke into a weak force acting on another moving element having a long stroke; i.e. the opposite function of the above-defined alternative mechanism of a normal servo mechanism. A first closed hydraulic system that controls another closed hydraulic system in which hydraulic means of the adjustment device is incorporated may be used. Minor changes in the amount of fluid in a smaller reservoir of the first system could then be transferred by the reverse servo into major changes in the amount of fluid in a larger reservoir in the second system. In consequence, the change of volume in the larger reservoir of the second system will affect the hydraulic means of the adjustment device. For example, a short stroke that decreases the volume of the smaller reservoir will cause the larger reservoir to supply the adjustment device with a large amount of hydraulic fluid, which in turn results in a long mechanical adjustment stroke on the restriction device. The great advantage of using such a reverse servo is that the larger volume system could be placed inside the abdomen or retroperitoneum where there is more space and still it would be possible to use manual manipulation means of the smaller system subcutaneously. The smaller reservoir could be controlled directly or indirectly by a fluid supply means. The fluid supply means may include another small reservoir, which may be placed subcutaneously and may be activated by manual manipulation means. Both the servo and reverse servo may be used in connection with all of the various components and solutions described in the present specification.

Preferably, the reverse servo comprises hydraulic means and a main fluid supply reservoir and eventually an additional fluid supply reservoir. Both reservoirs define a chamber containing hydraulic fluid, and the hydraulic means comprises first and second wall portions of the main fluid supply reservoir, which are displaceable relative to each other to change the volume of the chamber of the main fluid supply reservoir. The hydraulic means may control the adjustment device indirectly, e.g. via an increased amount of fluid in the main fluid supply reservoir, in response to a predetermined first displacement of the first wall portion of any of the reservoirs relative to the second wall portion of the reservoir to restrict the blood flow leaving the penis, and to control the adjustment device in response to a second displacement of the first wall portion of any reservoir relative to the second wall portion, to indirectly adjust the restriction device to release the penile tissue. The wall portions of the reservoirs may be designed to be displaceable relative to each other by manual manipulation thereof or be displaceable relative to each other by manually pushing, pulling or rotating any of the wall portions of the reservoir in one direction. Alternatively, the wall portions of the main fluid supply reservoir may be displaceable relative to each other by magnetic means, hydraulic means or electric control means including an electric motor.

The magnetic means, hydraulic means, or electrical control means may all be activated by manually manipulated means preferably located subcutaneously. This control may be indirect for example via a switch.

Even in the broadest embodiment of the invention the adjustment device may comprise a servo means. The servo means may comprise a hydraulic operation means, an electrical control means, a magnetic means, mechanical means or a manual manipulation means. The hydraulic operation means, electrical control means, mechanical means or magnetic means may be activated by manual manipulating means. Using a servo system will save the use of force when adjusting the adjustment device which may be of importance in many applications, for example when a battery cannot put out enough current although the total energy in the battery is more than enough to power the system.

In accordance with a preferred embodiment of the invention, the apparatus comprises implantable electrical components including at least one, or only one single voltage level guard and a capacitor or accumulator, wherein the charge and discharge of the capacitor or accumulator is controlled by use of the voltage level guard. As a result, there is no need for any implanted current detector and/or charge level detector for the control of the capacitor, which makes the apparatus simple and reliable.

All solutions may be controlled by a wireless remote control for controlling the adjustment device. The remote control may advantageously be capable of obtaining information related to the blood flow leaving the penis or the blood pressure or other important physical parameters and of commanding the adjustment device to adjust the restriction device in response to obtained information. With the wireless remote control the apparatus of the invention is conveniently controlled by the patient when he so desires, which is of great advantage compared to the prior art procedures. With the remote control the apparatus of the invention is conveniently controlled to adjust the implanted restriction device, which controls the blood flow leaving the penis. The restriction device may be operable to open and close the blood flow passageway formed by the penile exit veins. The restriction device may steplessly control the cross-sectional area of the passageway.

The apparatus may further comprise a pressure sensor for directly or indirectly sensing the pressure against the restriction device and the restriction device may control the blood flow in response to signals from the pressure sensor. The pressure sensor may be any suitable known or conventional pressure sensor such as shown in U.S. patents 5540731, 4846181, 4738267, 4571749, 4407296 or 3939823; or an NPC-102 Medical Angioplasty Sensor. The adjustment device preferaby non-invasively adjusts the restriction device to change the size of the cross-sectional area.

The adjustment device and/or other energy consuming components of the apparatus may be energised with wirelessly transmitted energy from outside the patient's body or be powered by an implanted battery or accumulator.

The apparatus may further comprise an implanted energy transforming device for transforming wireless energy directly or indirectly into kinetic energy for operation of the restriction device. The energy transforming device may, preferably directly, transform the wireless energy in the form of sound waves into electric energy for operation of the restriction device. Suitably the energy transforming device comprises a capacitor adapted to produce electric pulses from the transformed electric energy.

The apparatus of the invention may further comprise an energy transfer means for wireless transfer of energy from outside the patient's body to the adjustment device and/or other energy consuming implantable components of the apparatus. The energy transfer means may be adapted to intermittently transfer the energy, preferably electric energy, in the form of a train of energy pulses for direct use in connection with the energising of the energy consuming components of the apparatus. An implanted capacitor having a capacity less than 0,1 µF may be used for producing the train of energy pulses.

A motor may be implanted for operating the adjustment device, wherein the energy transfer means is adapted to directly power the motor with transferred energy. Alternatively, or in combination with the motor, a pump may be implanted for operating the adjustment device, wherein the energy transfer means is adapted to transfer wireless energy in the form of electromagnetic waves for direct power of the pump. Preferably, the pump is not a plunger type of pump, but may comprise a peristaltic or membrane pump.

The energy transfer means preferably transfers wireless energy in the form of electromagnetic waves. However, for safety radio waves may be excluded.

Alternatively, the energy transferred by the energy transfer means may comprise an electric field or a magnetic field.

Most preferred, the energy transferred by the energy transfer means comprises a signal.

Preferably, the wireless remote control comprises a separate signal transmitter or receiver and a signal receiver or transmitter implanted in the patient. For example, the signal transmitter and signal receiver may transmit and receive a signal in the form of digital pulses, which may comprise a magnetic or electric field. Alternatively, which is preferred, the signal transmitter and signal receiver may transmit and receive an electromagnetic wave signal, a sound wave signal or a carrier wave signal for a remote control signal. The receiver may comprise an implanted control unit for controlling the adjustment device in response to a control signal from the signal transmitter.

The apparatus of the invention may further comprise an implanted energiser unit for providing energy to energy consuming implanted components of the apparatus, such as electronic circuits and/or a motor for operating the adjustment device. The apparatus may comprise an external energy transmitter for transmitting wireless energy, wherein the energiser unit is adapted to transform the wireless energy into electric energy. An implanted electric motor may operate the adjustment device and the energiser unit may be adapted to power the electric motor with the electric energy transformed from the wireless energy.

The energiser unit may comprise a battery and a switch operable by the wireless energy transmitted by the external transmitter, for connecting the battery to the implanted energy consuming components of the apparatus in an "on" mode when the switch is powered by the wireless energy and to keep the battery disconnected from the energy consuming components in a "standby" mode when the switch is not powered.

The control unit may power such an implanted motor with energy provided by the energiser unit in response to a control signal received from the signal transmitter. Any known or conventional signal transmitter or signal receiver that is suitable for use with a human or mammal patient may be provided as the signal transmitter or signal receiver of the invention.

Generally, all the signals mentioned above may comprise electromagnetic waves, such as infrared light, visible light, laser light, micro waves, or sound waves, such as ultrasonic waves or infrasonic waves, or any other type of wave signals. The signals may also comprise electric or magnetic fields, or pulses. All of the above-mentioned signals may comprise digital signals. The control signals may be carried by a carrier wave signal, which in an alternative embodiment may be the same signal as the wireless energy signal. Preferably a digital control signal may be carried by an electromagnetic wave signal. The carrier wave or control signal may be amplitude or frequency modulated.

The motor may be any type of motor, such as a pneumatic, hydraulic or electric motor and the energiser unit may power the motor with pressurized gas or liquid, or electric energy, depending on the type of motor. Where the motor is an electric motor, it may power pneumatic or hydraulic equipment.

The energiser unit may comprise a power supply and the control unit may power the motor with energy from the power supply. Preferably, the power supply is an electric power supply, such as a battery, and the motor is an electric motor. In this case, the battery also continuously powers at least part of the circuitry of the signal receiver in a standby mode between the adjustments, in order to keep the signal receiver prepared for receiving signals transmitted from the signal transmitter.

The energiser unit may transfer energy from the control signal, as the control signal is transmitted to the signal receiver, into electric energy for powering the implanted electronic components. For example, the energiser unit may transfer the energy from the control signal into a direct or alternating current.

In case there is an implanted electric motor for operating the adjustment device the energiser unit may also power the motor with the transferred energy. Advantageously, the control unit directly powers the electric motor with electric energy, as the energiser unit transfers the signal energy into the electric energy. This embodiment is particularly simple and does not require any recurrent invasive measures for exchanging empty power supplies, such as batteries, that is required in the first embodiment described above. The motor may also be directly powered with wirelessly transmitted electromagnetic or magnetic energy in the form of signals, as the energy is transmitted. All the various functions of the motor and associated components described in the present specification may be used where applicable.

For adjustment devices of the type that requires more, but still relatively low, power for its operation, the energiser unit may comprise a rechargeable electric power supply for storing the electric energy obtained and the control unit may power the electric motor with energy from the rechargeable electric power supply in response to a control signal received from the signal transmitter. In this case, the rechargeable power supply can be charged over a relatively long time (e.g. a few seconds up to a half hour) without powering the electric motor.

The electric power supply suitably comprises an inexpensive simple capacitor. In this case, the electric motor may be a stepping motor. In all embodiments the motor may preferable be able to perform a reversing function.

The signal transmitter may transmit an electromagnetic signal and the energiser unit may draw radiant energy from the electromagnetic wave signal, as the latter is transmitted to the signal receiver, and transfer the radiant energy into electric energy.

Alternatively, the energiser unit may comprise a battery or accumulator, an electrically operable switch adapted to connect the battery to the signal receiver in an on mode when the switch is powered and to keep the battery disconnected from the signal receiver in a standby mode when the switch is not powered, and a rechargeable electric power supply for powering the switch. The control unit may power the electric motor with energy from the battery in response to a control signal received from the signal transmitter, when the switch is in its on mode. Advantageously, the energiser unit may transform wave energy from the control signal, as the latter is transmitted to the signal receiver, into a current for charging the rechargeable electric power supply, which suitably is a capacitor. Energy from the power supply is then used to change the switch from off (standby mode) to on. This embodiment is suited for adjustment devices of the type that require relatively high power for their operation and has the advantage that the electronic circuitry of the signal receiver does not have to be powered by the battery between adjustments. As a result, the lifetime of the battery can be significantly prolonged. The switch may be switched manually or by the use of magnetic or electric energy.

As an example, the signal transmitter may transmit an electromagnetic wave signal and the energiser unit may draw radiant energy from the electromagnetic wave signal, as the latter is transmitted to the signal receiver, and may transfer the radiant energy into said current. The energiser unit suitably comprises a coil of the signal receiver for inducing an alternating current as the electromagnetic wave signal is transmitted through the coil and a rectifier for rectifying the alternating current. The rectified current is used for charging the rechargeable power source.

Alternatively, the signal transmitter and receiver may solely be used for a control signal and a further pair of signal transmitter and receiver may be provided for transferring signal energy to implanted components. By such a double system of signal transmitters and receivers the advantage is obtained that the two systems can be designed optimally for their respective purposes, namely to transmit a control signal and to transfer energy from an energy signal. Accordingly, the apparatus may further comprise an external energy transmitter for transmitting wireless energy, wherein the energiser unit comprises a battery and an operable switch for connecting the battery to the signal receiver in an "on" mode when the switch is powered and for keeping the battery disconnected from the signal receiver in a "standby" mode when the switch is not powered, and the external energy transmitter powers the switch. Suitably, the energy transmitter may directly power the switch with the wireless energy to switch into the "on" mode. As should be realized by a skilled person, in many of the above-described embodiments of the invention the adjustment device may be operated by control means or manual manipulation means implanted under the skin of the patient, such as a pump, an electrical switch or a mechanical movement transferring means. In the manual embodiment it is not necessary to use a motor for operating the adjustment device.

In embodiments including hydraulic transmission means, an injection port connected to the hydraulic means may be provided for enabling, normally single, once-and-for-all, calibration of the amount of fluid in the hydraulic system.

In all embodiments a motor may be operatively connected to the adjustment device. A reversing device may be implanted in the patient for reversing the motor. The adjustment device preferably adjusts the restriction device in a non-manual manner without the patient' touching his skin.

The adjustment device may be adapted to hydraulically adjust the restriction device by using hydraulic means which is devoid of hydraulic fluid of the kind having a viscosity that substantially increases when exposed to heat or a magnetic field, *i.e.* the hydraulic fluid would not become more viscous when exposed to heat or influenced by magnetic forces.

All the above-described various components, such as the motor, pump and capacitor, may be combined in the different embodiments where applicable. Also the various functions described in connection with the above embodiments of the invention may be used in different applications, where applicable.

All the various ways of transferring energy and controlling the energy presented in the present specification may be practised by using all of the various components and solutions described.

The invention may be used in a method for treating male sexual impotence, comprising surgically implanting in the body of a male patient suffering from sexual impotence an adjustable restriction device which directly engages a portion of the normal penile tissue of the patient to affect the blood flow leaving the penis and the prolongation thereof, and when desired to achieve erection, mechanically adjusting the restriction device to temporarily restrict the blood flow leaving the penis.

The method may further comprise implanting the male sexual impotence treatment apparatus in the base of the patient's penis or its prolongation, preferably implanting an restriction device engaging the corpora cavernosa, crura or the prolongation thereof as a single unit or engaging the two corpora cavernosa or crura or the prolongations thereof separately. The method may comprise implanting the restriction device engaging one or both of the crura of the penis.

As a modification, the method may further comprise implanting the restriction device engaging at least one of the exit veins from the penis.

The invention provides another method for treating male sexual impotence, comprising the steps of: placing at least two laparascopical trocars in the body of a male patient suffering from sexual impotenc, inserting a dissecting tool through the trocars and dissecting an area of the penis and abdominal or peritoneal surroundings, placing at least one adjustable restriction device in the dissected area engaging the penile tissue or the prolongation thereof, and adjusting the restriction device to restrict the blood flow leaving the penis when the patient wishes to achieve erection.

The method may further comprise mechanically adjusting said restriction device in a non-manual manner. The restriction device may engage (a) both of the corpora cavernosa, the crura of the penis or the prolongation thereof as a single unit; or (b) one of the exit veins from the penis.

Alternatively, the method may further comprise implanting (a) a further adjustable restriction device, wherein the two restriction devices engage the two corpora cavernosa, the crura of the penis or their prolongations, respectively, as separate units; or (b) several restriction devices engaging respective exit veins from the penis.

The method may further comprise implanting a source of energy in the patient and providing a control device for controlling the source of energy from outside the patient's body to supply energy to the restriction device.

The invention is described in more detail in the following with reference to the accompanying drawings, in which
FIGURE 1 is a schematic sectional view of a preferred first embodiment of the male sexual impotence treatment apparatus in accordance with the invention;
FIGURES 2 and 3 are cross-sectional views taken along the lines II-II and III-III, respectively, of FIGURE 1;
FIGURES 4 and 5 schematically show two alternative designs of the embodiment of FIGURE 1;
FIGURE 6 schematically illustrates a motor arrangement for the design according to FIGURE 5;
FIGURE 7 is a schematic sectional view of a second embodiment of the apparatus in accordance with the invention;
FIGURE 8 schematically illustrates a hydraulic transmision conduit for the embodiment of FIGURE 7;
FIGURE 9 is a schematic sectional view of a third embodiment of the apparatus in accordance with the invention;
FIGURE 10 is a modification of the embodiment of FIGURE 9;
FIGURE 11 is a schematic view of a fourth embodiment of the apparatus in accordance with the invention;
FIGURES 12 and 13 are enlarged details of the embodiment of FIGURE 11;
FIGURE 14 is a cross-section along the line XIV-XIV of FIGURE 11;
FIGURE 15 is a schematic view of a fifth embodiment of the apparatus in accordance with the invention;
FIGURE 16 is an enlarged detail of FIGURE 15;.
FIGURE 17 is a cross-section along the line XVII-XVII of FIGURE 15;
FIGURES 18 to 21 are schematic sectional views of a sixth, seventh, eighth and ninth embodiments, respectively, of the apparatus in accordance with the invention;
FIGURES 22 and 23 illustrate a fully open and a reduced restriction opening, respectively, of the embodiment of FIGURE 21;
FIGURE 24 is a schematic view of a tenth embodiment of the apparatus in accordance with the invention;
FIGURE 25 is an enlarged detail of the embodiment of FIGURE 24;
FIGURES 26 and 27 illustrate a fully open and a reduced restriction opening, respectively, of the embodiment of FIGURE 24;
FIGURE 28 schematically illustrates a cushion arrangement for protecting the tissue of the patient;
FIGURE 29A-D is a block diagram of four different principal embodiments of the invention;
FIGURE 30A-D are cross-sectional views of a pump mechanism according to FIGURE 29C, which pumps fluid in opposite directions by mechanically pushing a wall portion in only one direction;
FIGURE 31 is a cross-sectional view of a reservoir having a variable volume controlled by a remote control motor, in accordance with a particular embodiment of the principal embodiment shown in FIGURE 29B or 30B;
FIGURE 32 is a cross-sectional view of a reservoir having a variable volume adjustable by manual manipulation, in accordance with a particular embodiment of the principal embodiment shown in FIGURE 29B or 29D;
FIGURE 33A is a front view of a hydraulic, pneumatic or mechanical reverse servo system in accordance with a particular embodiment of the principal embodiment shown in FIGURE 29D;
FIGURE 33B is a cross-sectional view taken along line VB-VB of FIGURE 33A;
FIGURE 34 is a block diagram illustrating remote control components of the apparatus of the invention;
FIGURE 35 is a schematic view of a circuitry used for the system of the block diagram of FIGURE 34;
FIGURES 36A and 36B are schematic views of an eleventh embodiment of the apparatus in accordance with the invention;
FIGURES 37A and 37B are schematic views of a twelfth embodiment of the apparatus in accordance with the invention;
FIGURE 38 is a schematic view of a thirteenth embodiment of the apparatus in accordance with the invention;
FIGURES 39A, 39B and 39C are a schematic front view and schematic sectional views, respectively, of a fourteenth embodiment of the apparatus in accordance with the invention;
FIGURES 40A through 44B are five modifications of the embodiment of FIGURES 39A-39C;
FIGURE 45 illustrates the apparatus of the invention with a restriction device implanted around the corpus cavernosum of a patient; and
FIGURE 46 illustrates the apparatus of the invention with two restriction members implanted around respective exit veins from the penis of a patient.

Referring to the drawing figures, like reference numerals designate identical or corresponding elements throughout the several figures.

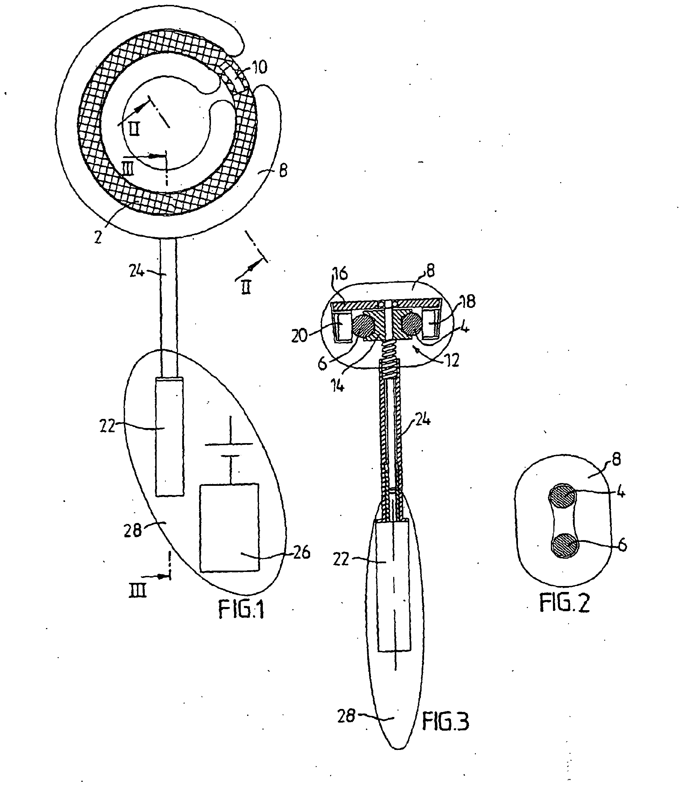

FIGURES 1-3 show a preferred embodiment of the male sexual impotence treatment apparatus of the invention comprising a restriction device having an elongated restriction member in the form of a circular resilient core 2 with two overlapping end portions 4,6. The core 2 defines a substantially circular restriction opening and is enclosed in an elastic soft hose 8 except at a releasable and lockable joint 10 of the core 2, which when released enables application of the core 2 with its hose 8 around a tissue of a patient, such as the corpus cavernosum or one or more exit veins from the patient's penis. The materials of all of these elements are bio-compatible so that the patient's body will not reject them. A mechanical adjustment device 12 for mechanically adjusting the longitudinal extension of the core 2 to change the size of the restriction opening comprises a drive wheel 14 in frictional engagement with the overlapping end portions 4,6 of the core 2. The drive wheel 14 is journalled on a holder 16 placed in the hose 8 and provided with two counter pressure rollers 18,20 pressing the respective end portions 4, 6 of the core 2 against the drive wheel 14 to increase the frictional engagement therebetween. An electric motor 22 is connected to the drive wheel 14 via a long flexible drive shaft 24 and is moulded together with a remote controlled power supply unit 26 in a body 28 of silicone rubber. The length of the flexible drive shaft 34 is selected so that the body 28 can be placed in a desired position in the patient's body, suitably in the abdomen.

When the patient wishes to achieve erection, he controls the power supply unit 26 to power the electric motor 22 to turn the drive wheel 14 in one direction to reduce the diameter of the core 2, so that the penile tissue is squeezed and the blood flow leaving the penis is restricted. When the patient wishes to regain flaccid condition of his penis, he controls the power supply unit 26 to power the electric motor 22 to turn the drive wheel 14 in the opposite direction to increase the diameter of the core 2, so that the tissue is released.

Alternatively, a rack gear may be formed on one of the end portions 4,6 of the core 2 and the drive wheel 14 may be replaced by a drive gear wheel connected to the other end portion of the core 2 and in mesh with the rack gear.

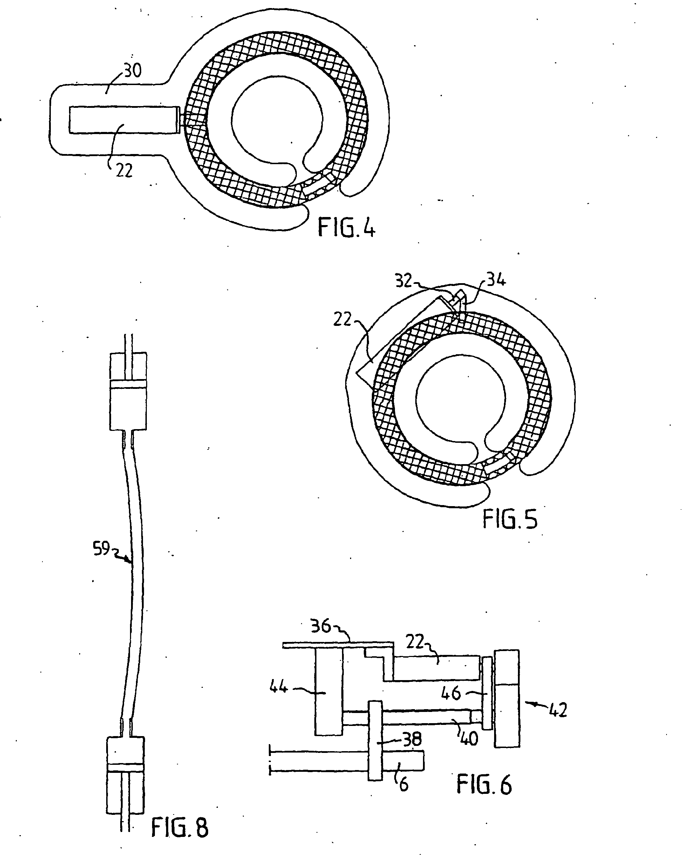

FIGURE4 shows an embodiment of the invention which is identical to the embodiment of FIGURES. 1-3, except that the motor 22 is encapsulated in a lateral protrusion 30 of the hose 8 so that it is fixed to the core 2 and has a short drive shaft 32 onto which the drive wheel 14 is mounted, and that the motor 22 is positioned relative to the circular core 2 such that the drive shaft 32 extends radially thereto.

FIGURE 5 shows an embodiment of the invention which likewise is identical to the embodiment of FIGURES 1-3, except that the motor 22 is encapsulated in the hose 8 so that it is fixed to the core 2 and has a short drive shaft 32, and that the motor 22 is positioned relative to the core 2 such that the drive shaft 32 extends substantially tangentially to the circular core 2. There is an angular gearing 34 connecting the drive shaft 32 to the drive wheel 14.

FIGURE 6 shows a suitable arrangement for the motor 22 in the embodiment of FIGURE 5, comprising a first clamping member 36 secured to one end portion of the core 2 and a second clamping member 38 secured to the other end portion 6 of the core 2. The motor 22 is secured to the first clamping member 36 and is operatively connected to a worm 40 via a gear transmission 42. The worm 40 is journalled at its opposite ends on holders 44 and 46, which are- rigidly secured to the clamping member 36 and the motor 22, respectively. The second clamping member 38 has a pinion in mesh with the worm 40. When the motor 22 is powered the worm 40 rotates and will thereby pull the end portion 6 of the core 2 in one or the opposite longitudinal direction, so that the diameter of the substantially circular core 2 is either increased or decreased.

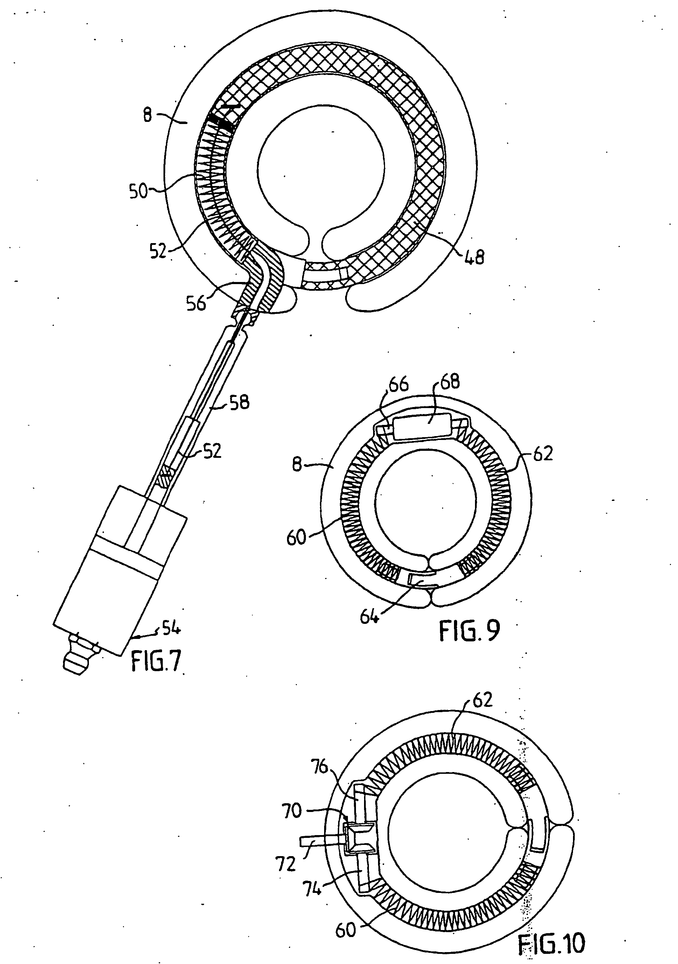

FIGURE 7 shows an embodiment of the invention in which the elongated restriction member comprises a core. 48 and a helical spring 50. A spring contracting means in the form of a flexible pulling member 52, *i.e.* a string, wire or cable, is connected to the core 48 at one end thereof and extends through the helical spring 50. A hydralic motor in the form of a cylinder/piston unit 54 is adapted to pull the flexible pulling member 52 to contract the helical spring 50 against an arresting member 56, which is fixed relative to the core 48. A tube 58 hinged to the arresting member 56 extends between the cylinder/piston unit 54 and the arresting member 56, the flexible pulling member 52 running through the tube 58 and being connected to the piston of the cylinder/piston unit 54. FIGURE B shows a similar embodiment in which a hydraulic transmission conduit 59 is provided between two piston-cylinder assemblies 54, for use as the hydraulic motor/device in FIGURE 7.

FIGURE 9 shows an embodiment of the invention in which the restriction member comprises two elongated helical springs 60 and 62 having free ends, and a body 64 to which the springs 60,62 are nonrotatably secured at their opposite ends. The body 64 comprises two separate parts secured to opposite end portions of the enclosing elastic hose 8 and is designed with a releasable and lockable joint between the separate parts. An adjustment device in the form of a drive shaft 66 has two opposite end portions connected to the helical springs 60,62, respectively, at their free ends. The coils of the springs 60, 62 form left and right hand helices, respectively. A motor 68 is adapted to rotate the drive shaft 66 in one direction to enlarge the coils of the helical springs 60,62 to longitudinally contract the springs 60,62 and to rotate the drive shaft 66 in the opposite direction to reduce the size of the coils of the springs 60,62 to longitudinally extend the springs 60,62. Thus, the elongated helical springs 60, 62 defines a restriction opening, the size of which is increased when the springs 60,62 are extended and decreased when the springs 60,62 are contracted.

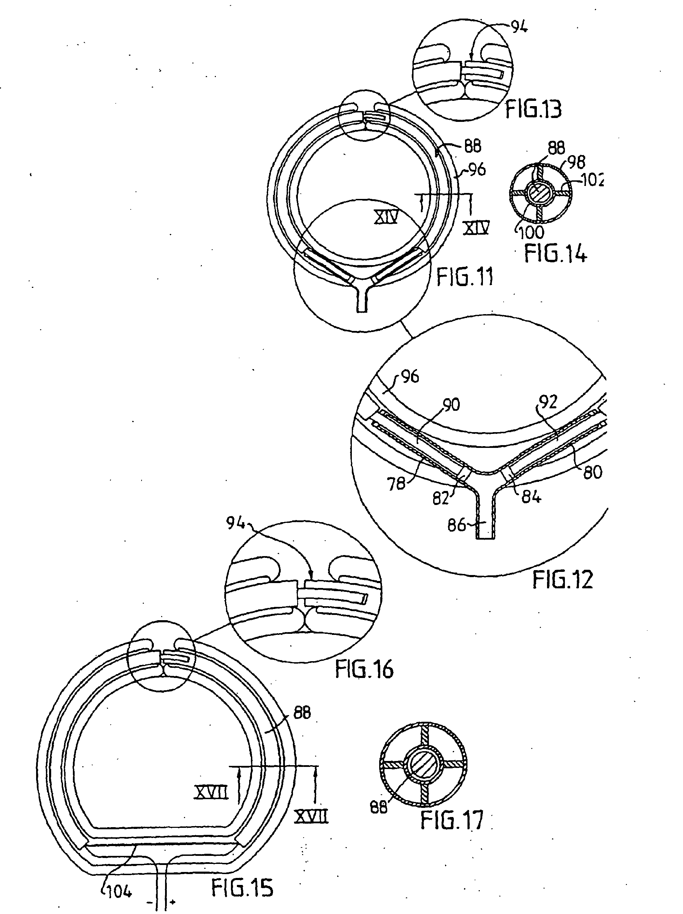

FIGURE 10 shows an embodiment according to the invention which is identical.to the embodiment of FIGURE 9, except that the adjustment decice comprises a gearing having an input shaft 72 and two opposite aligned output shafts 74 and 76 connected to the helical springs 60 and 62, respectively, at their free ends. The input shaft 72 is connected to the output shafts 74,76 such that they rotate at opposite directions upon rotation of the input shaft 72. The coils of the springs 60, 62 form the same helices.

FIGURES 11-14 show an embodiment of the device of the invention in which a hydraulic motor comprises two interconnected cylinders 78 and 80 and two pistons 82 and 84 in the respective cylinders 78,80. The cylinders 78,80 have a common fluid supply inlet member 86, which together with the cylinders 78,80 takes the shape of a Y-pipe. The restriction member comprises an elongated resilient arcuate core 88. The adjustment device comprises two bars 90 and 92 secured to opposite ends of the core 88 and connected to the pistons. 82 and 84, respectively. The core 88 defines a restriction opening and is provided with a releasable and lockable joint 94 (FIGURE 13) to permit application of the core 88 around the tissue. The core 88 and the cylinders 90, 92 are enclosed by a soft elastic hose 96 except at the joint 94 and the inlet member 86. The hose 96 has an outer tubular wall 98 and a central coaxial inner tubular wall 100, which is fixed to the outer wall 98 by spoke members 102 (FIGURE 14). The core 88 is loosely fit in the inner tubular wall 100. By supplying fluid to or withdrawing fluid from the inlet 86 the pistons 82 and 84 will move towards or from each other, so that the restriction opening defined by the core 88 is changed by the longitudinal displacement of' the bars 90,92.

FIGURES 15-17 show an embodiment of the invention which is identical to the embodiment of FIGURES 11-14, except that the adjustment device comprises an elongated voltage responsive element 104 secured to the opposite ends of the core 88, so that the core 88 and the element 104 form the restriction member. The element 104 is capable of bending inwardly into a bow in response to a voltage applied across the element 104. The radius of curvature of said bow is adjustable by changing the level of the voltage applied to element 104.

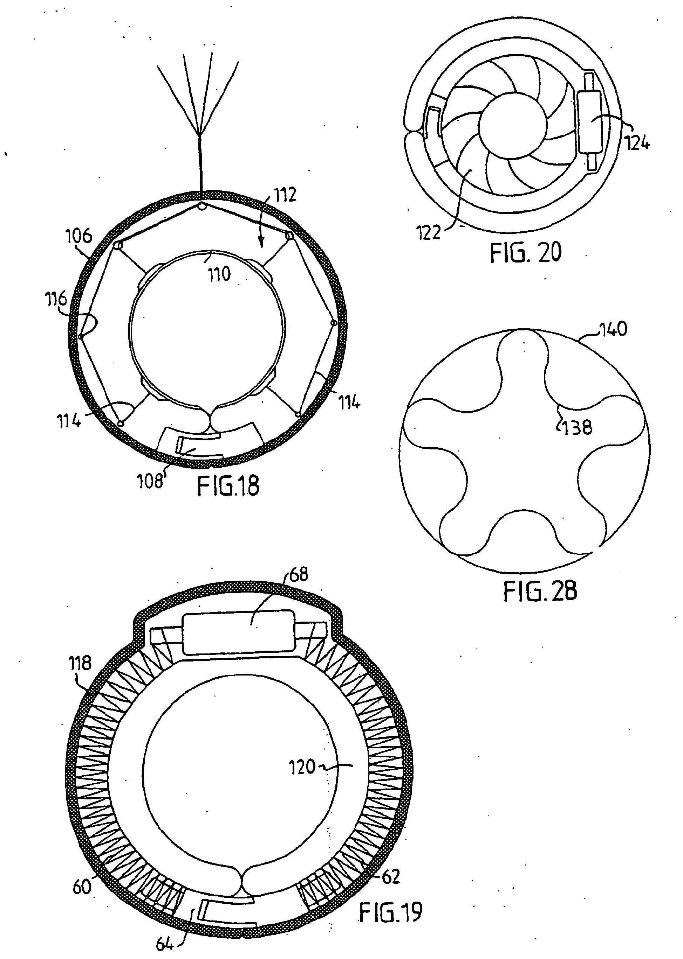

FIGURE 18 shows an embodiment of the invention comprising a loop forming means in the form of a substantially rigid outer circular element 106 with a releasable and lockable joint 108. In this embodiment the restriction member comprises an elastic inner circular element 110 formed by the innermost wall portion of an elastic hose 112 extending along the outer element 106. The inner circular element 110 is disposed concentrically within the outer circular element 106. The adjustment device comprises a plurality of threads 114 secured to the elastic inner element 110 along the circumference thereof and running from the inner element 110 via guide members 116 attached to the outer element 106. By pulling all the threads 114 the inner elastic element 110 is pulled under expansion radially outwardly towards the outer element 106.

FIGURE 19 shows an embodiment which is identical to the embodiment of FIGURE, 9, except that it comprises a loop forming means in the form of a substantially rigid outer circular element 118 supporting the helical springs 60, 62, and a soft elastic inner wall 120 extending along the springs 60,62. When the motor 68 rotates the helical springs 60, 62 in a direction that enlarges the coils of the springs 60,62, the coils are forced by the rigid outer element 118 to expand radially inwardly thereby reducing the size of the restriction opening formed by the circumferential confinement surface of the restriction member (springs 60,62 and body 64).

FIGURE 20 shows an embodiment of the invention in which a restriction member comprises a plurality of arcuate lamellae 122 arranged like the conventional adjustable aperture mechanism of a camera. The adjustment device, not shown, is conventional and is operated by a motor 124 to adjust the lamellae 122 to change the size of an restriction opening defined by the lamellae 122.

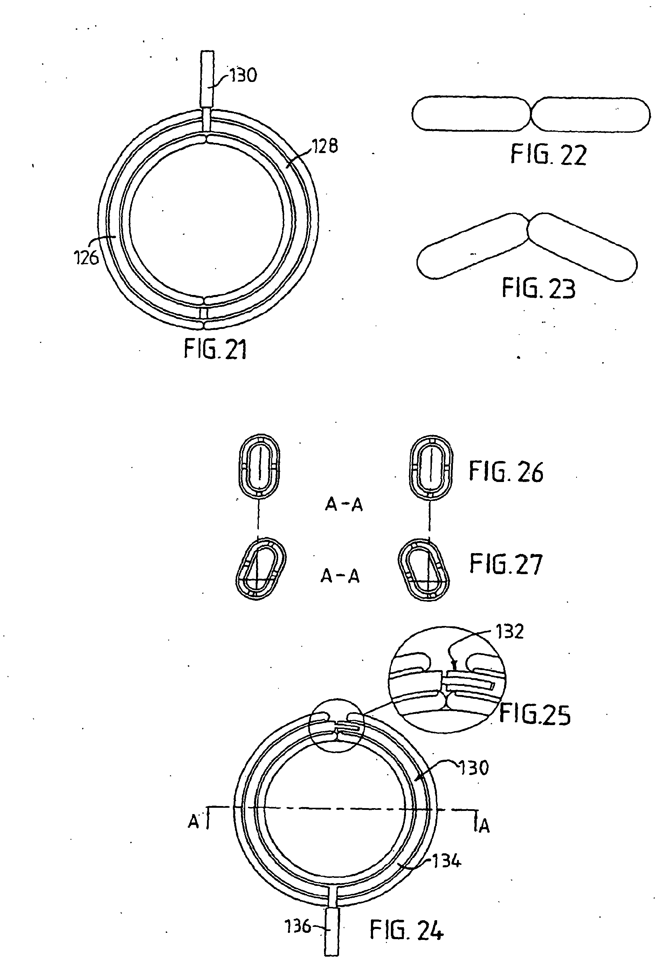

FIGURES 21-23 show an embodiment of the invention in which a restriction member comprises two semi-circular elements 126 and 128 which are hinged together such that the semi-circular elements 126,128 are swingable relative to each other between a fully open state in which they substantially form a circle, illustrated in FIGURE, 22 and an angular state, in which the size of the restriction opening defined by the semi-circular elements 126,128 is reduced, illustrated in FIGURE, 23. The adjustment device, not shown, is conventional and is operated by a motor 130 to swing the semi-circular elements 126,128 relative to each other.

FIGURES 24-27 show an embodiment of the invention in which a restriction member comprises an elastic belt 130 forming a circle and having a substantially oval cross-section. The restriction member 130 is provided with a releasable and lockable joint 132. An elastic double walled hose 134 encloses the belt 130 except at the joint 132. The adjustment device, not shown, is conventional and is operated by a motor 136 to turn the belt 130 around the longitudinal extension thereof between a fully open state, in which the inner broader side of the belt 130 forms a substantially cylindrical surface, illustrated in FIGURE 26, and a reduced open state, in which the inner broader side of the belt 130 forms a substantially conical surface, illustrated in FIGURE 27.

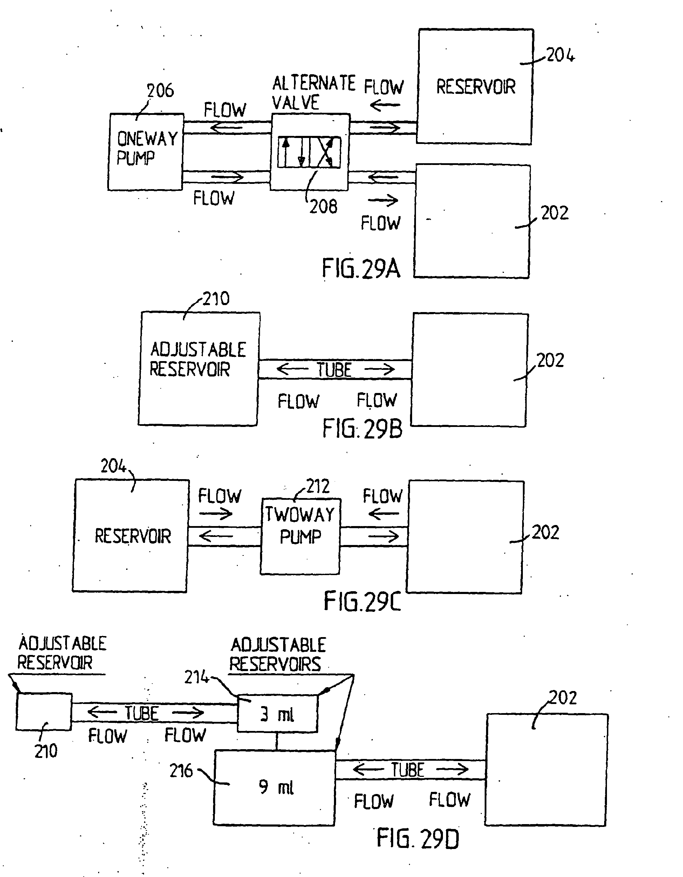

FIGURE 28 schematically illustrates a cushion arrangement for protecting the penile tissue, comprising a plurality of cushions 138 disposed in series along a substantially circular holding member 140. This cushion arrangement may be utilized in any of the above described embodiments of the invention.

FIGURES 29A-D provide a block diagram of four different hydraulic transmission configurations. FIGURE 29A shows an adjustment device 202, a separate reservoir 204, a one way pump 206 and an alternate valve 208. FIGURE 29B shows the adjustment device 202 and an adjustable reservoir 210. FIGURE 29C shows the adjustment device 202, a two-way pump 212 and the reservoir 204. FIGURE 29D shows a reverse servo system with a first closed system controlling a second system. The servo system comprises an adjustable servo reservoir 210 and a passive adjustable fluid supply reservoir 214. Any of the reservoirs can be the active reservoir, either the servo reservoir 210 or the fluid supply reservoir 214. The reservoir 214 controls a larger adjustable reservoir 216, which is used for the operation of the adjustment device 202 for changing the restriction opening of the restriction member.

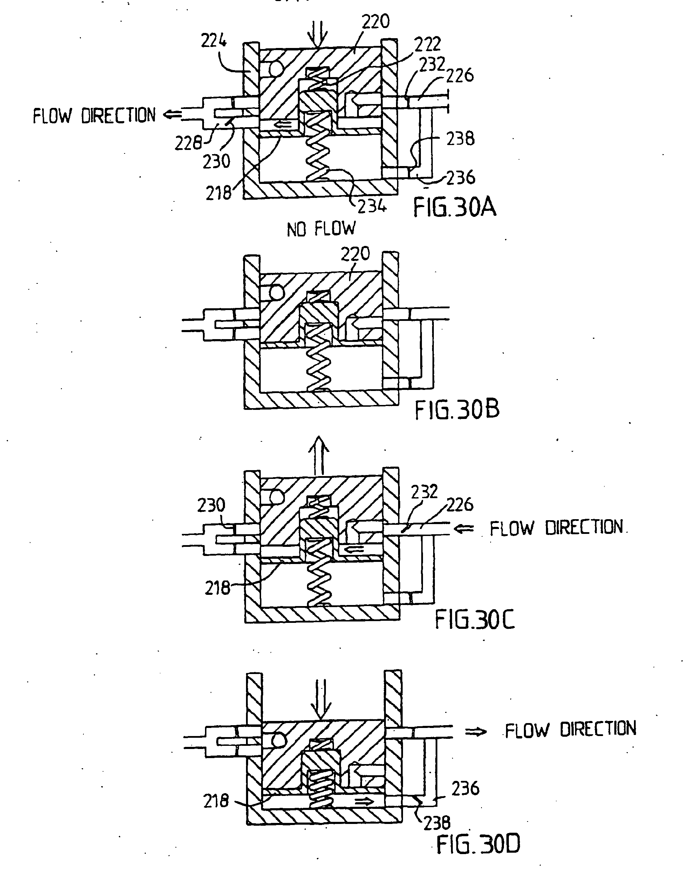

FIGURES 30A-D are cross-sectional views of a pump mechanism adapted to pump fluid in both directions only by mechanically pushing a separate sealing wall portion 218 in one direction. FIGURE 30A shows a piston 220 pushed forwards against a spring 222 towards the wall portion 218 and located in a pump housing 224 conducting fluid from a right upper fluid passage 226 of the housing 224 to a left fluid passage 228 of the housing 224. A main valve 230 is open and a nonreturn valve 232 is closed. FIGURE 30B illustrates the first pump movement in which the piston 220 has moved forwards and reaches the wall portion 218. FIGURE 30C illustrates how the piston 220 moves backwards by the action of the spring 222. The main valve 230 is now closed and the nonreturn valve 232 is open for fluid from the right upper passage 226. FIGURE 30D illustrates how the piston 220 is moved further downwards from its position according to FIGURE 30B while pushing the wall portion 218 downwards against a second spring 234 that is stronger than spring 222, so that fluid escapes from a right lower fluid passage 236. When moving the piston 220 backwards from the position of FIGURE 30D, fluid enters the left fluid passage 228 and a valve 238 in the lower right fluid passage 236 closes.

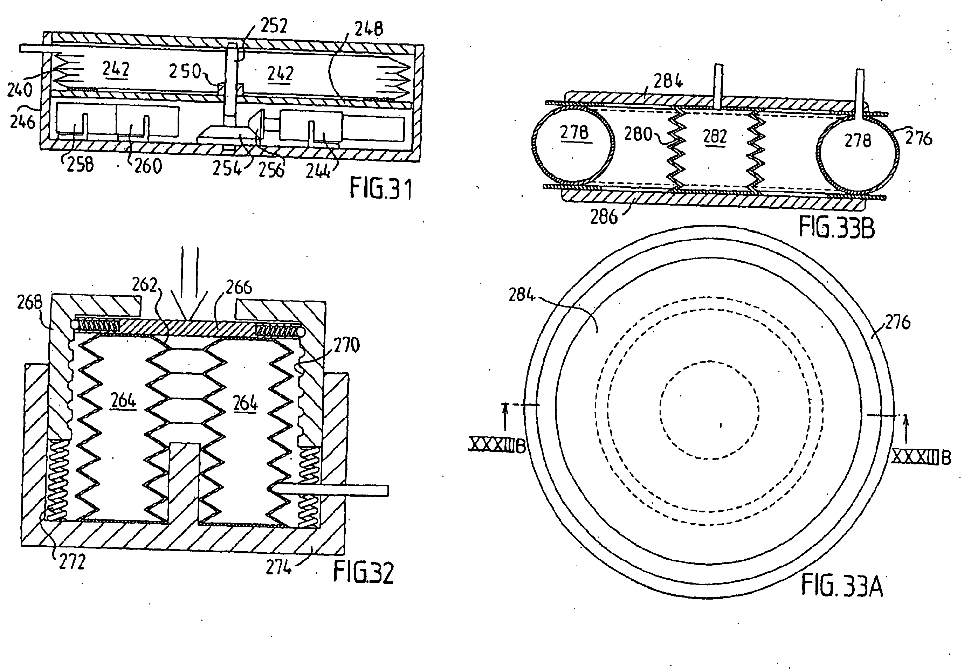

FIGURE 31 is a cross-sectional view of a reservoir 240 defining a chamber 242, the size of which is variable and is controlled by a remote controlled motor 244, in accordance with FIGURE 29B or 29D. The reservoir 240 and the motor 244 are placed in a housing 246. The chamber 242 is varied by moving a large wall 248. The wall 248 is secured to a nut 250, which is threaded on a rotatable spindle 252. The spindle 252 is rotated by the motor 244 via an angular gearing, which comprises two conical gear wheels 254 and 256 in mesh with each other. The motor 244 is powered by a battery 258 placed in the housing 246. A signal receiver 260 for controlling the motor 244 is also placed in the housing 246. Alternatively, the battery 258 and the signal receiver 260 may be mounted in a separate place. The signal receiver may comprise any known or conventional device which is capable of receiving a control signal and then operating the motor 244.

FIGURE 32 is a cross-sectional view of a reservoir 262 defining a chamber 264, the size of which is variable and is controlled by manual manipulation. A gable wall portion 266 of an open ended inner cylindrical housing 68 is adapted to be pushed downwards to fit in a desired locking groove 270 of a plurality of locking grooves 270 on the mantle wall of the cylindrical housing 268, to reduce the size of the chamber 64. The inner cylindrical housing 268 is suspended by springs 272 and is telescopically applied on an outer cylindrical housing 274. When pushing the inner cylindrical housing 268 it moves downwards relative to the outer cylindrical housing 274 causing the gable wall portion 266 to release from the locking groove 270 and move upwards relative to the inner cylindrical housing 268. When the inner housing 268 is moved upwardly by the action of the springs 272 the size of the chamber 264 is increased.

FIGURE 33A and 33B show a reverse servo means comprising a main ring-shaped fluid reservoir 276 defining a chamber 278, the size of which is variable. Centrally positioned in the main ring-shaped reservoir 276 there is a servo fluid reservoir 280 defining a chamber 282, the size of which is variable. The chamber 282 of the servo reservoir 280 is significantly smaller than the chamber 278 of the main reservoir 276. The two reservoirs 276 and .280 are situated between two opposite separate walls 284 and 286, and are secured thereto. When changing the amount of fluid in the servo reservoir 280, the two opposite walls 284, 286 are moved towards or away from each other, whereby the size of the chamber 278 of the main reservoir 276 is changed.

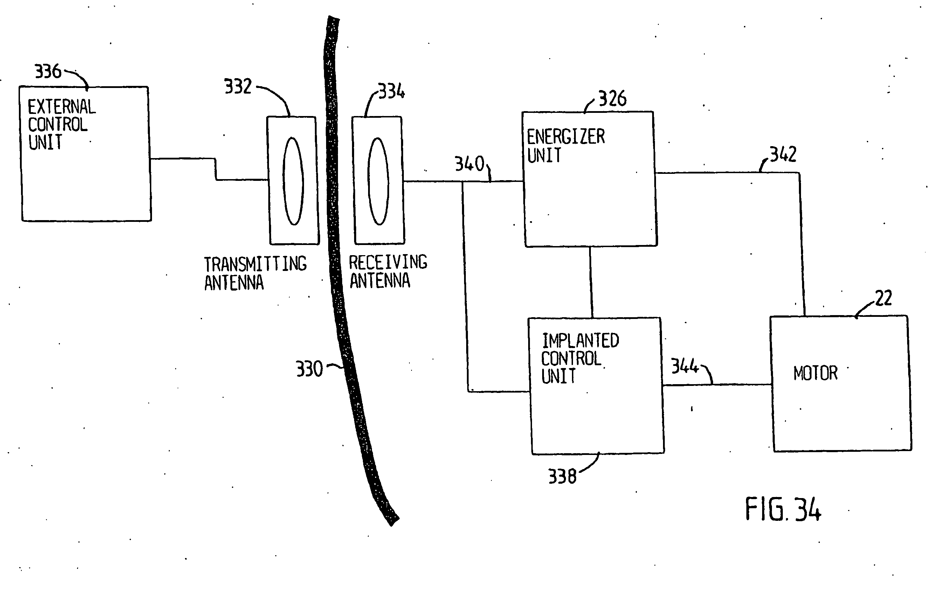

FIGURE 34 shows the basic parts of a remote control system of the apparatus of the invention including a motor, for instance the electric motor 22. In this case, the remote control system is based on the' transmission of an electromagnetic wave signal, often of a high frequency in the order of 100 kHz - 1 gHz, through the skin 330 of the patient. In FIGURE 34, all parts placed to the left of the skin 330 are located outside the patient's body and all parts placed to the right of the skin 330 are implanted in the patient's body.

An external signal transmitting antenna 332 is to be positioned close to a signal receiving antenna 334 implanted in the patient's body close to the skin 330. As an alternative, the receiving antenna 334 may be placed for example inside the abdomen of the patient. The receiving antenna 334 comprises a coil, approximately 1-100 mm, preferably 25 mm in diameter, wound with a very thin wire and tuned with a capacitor to a specific high frequency. A small coil is chosen if it is to be implanted under the skin of the patient and a large coil is chosen if it is to be implanted in the abdomen of the patient. The transmitting antenna 332 comprises a coil having about the same size as the coil of the receiving antenna 334 but wound with a thick wire that can handle the larger currents that is necessary. The coil of the transmitting antenna 332 is tuned to the same specific high frequency as the coil of the receiving antenna 334.

An external control unit 336 comprises a microprocessor, a high frequency electromagnetic signal generator and a power amplifier. The microprocessor of the control unit 336 is adapted to switch on/off the generator and to modulate signals generated by the generator to send digital information via the power amplifier and the antennas 332,334 to an implanted control unit 338. To avoid that accidental random high frequency fields trigger control commands, digital signal codes are used. A keypad placed on the external control unit 336 is connected to the microprocessor thereof. The keypad is used to order the microprocessor to send a digital signal to either increase or decrease the size of the restriction opening defined by the loop of the restriction member (*e.g.* as described above). The microprocessor starts a command by applying a high frequency signal on the antenna 332. After a short time, when the signal has energised the implanted parts of the control system, commands are sent to increase or decrease the size of the restriction opening of the restriction member in predefined steps. The commands are sent as digital packets in the form illustrated below.

| | | | |
|---|---|---|---|
| Start pattern, 8 bits | Command, 8 bits | Count, 8 bits | Checksum, 8 bits |

The commands are sent continuously during a rather long time period (*e.g.* 30 seconds or more). When a new increase or decrease step is desired the Count byte is increased by one to allow the implanted control unit 338 to decode and understand that another step is demanded by the external control unit 336. If any part of the digital packet is erroneous, its content is simply ignored.

Through a line 340, an implanted energiser unit 326 draws energy from the high frequency electromagnetic wave signal received by the receiving antenna 334. The energiser unit 326 stores the energy in a power supply, such as a large capacitor, powers the control unit 338 and powers the electric motor 22 via a line 342.

The control unit 338 comprises a demodulator and a microprocessor. The demodulator demodulates digital signals sent from the external control unit 336. The microprocessor of the control unit 338 receives the digital packet, decodes it and, provided that the power supply of the energiser unit 326 has sufficient energy stored, sends a signal via a signal line 344 to the motor 22 to either increase or decrease the size of the restriction opening of the restriction member depending on the received command code.

Alternatively, the energy stored in the power supply of the energiser unit may only be used for powering a switch, and the energy for powering the motor 22 may be obtained from another implanted power source of relatively high capacity, for example a battery. In this case the switch is adapted to connect the battery to the control unit 338 in an "on" mode when said switch is powered by said power supply and to keep said battery disconnected from the control unit in a "standby" mode when the switch is not powered.

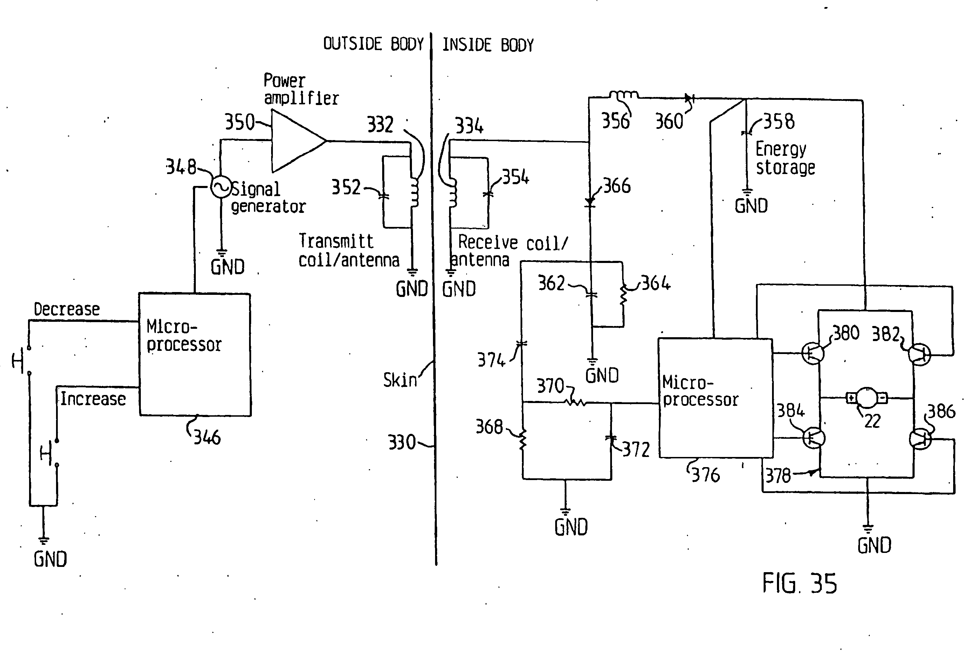

With reference to FIGURE 35, the remote control system schematically described above will now be described in accordance with a more detailed embodiment. The external control unit 336 comprises a microprocessor 346, a signal generator 348 and a power amplifier 350 connected thereto. The microprocessor 346 is adapted to switch the signal generator 348 on/off and to modulate signals generated by the signal generator 348 with digital commands that are sent to implanted components. of the device of the invention. The power amplifier 350 amplifies the signals and sends them to the external signal transmitting antenna 332. The antenna 332 is connected in parallel with a capacitor 352 to form a resonant circuit tuned to the frequency generated by the signal generator 348.

The implanted signal receiving antenna coil 334 forms together with a capacitor 354 a resonant circuit that is tuned to the same frequency as the transmitting antenna 332. The signal receiving antenna coil 334 induces a current from the received high frequency electromagnetic waves and a rectifying diode 360 rectifies the induced current, which charges a storage capacitor 358. A coil 356 connected between the antenna coil 334 and the diode 360 prevents the capacitor 358 and the diode 360 from loading the circuit of the signal receiving antenna 334 at higher frequencies. Thus, the coil 356 makes it possible to charge the capacitor 358 and to transmit digital information using amplitude modulation.

A capacitor 362 and a resistor 364 connected in parallel and a diode 366 forms a detector used to detect amplitude modulated digital information. A filter circuit is formed by a resistor 368 connected in series with a resistor 370 connected in series with a capacitor 372 connected in series with the resistor 368 via ground, and a capacitor 374, one terminal of which is connected between the resistors 368,370 and the other terminal of which is connected between the diode 366 and the circuit formed by the capacitor 362 and resistor 364. The filter circuit is used to filter out undesired low and high frequencies. The detected and filtered signals are fed to an implanted microprocessor 376 that decodes the digital information and controls the motor 22 via an H-bridge 378 comprising transistors 380,382,384 and 386. The motor 22 can be driven in two opposite directions by the H-bridge 378.

The microprocessor 376 also monitors the amount of stored energy in the storage capacitor 358. Before sending signals to activate the motor 22, the microprocessor 376 checks whether the energy stored in the storage capacitor 358 is enough. If the stored energy is not enough to perform the requested operation, the microprocessor 376 waits for the received signals to charge the storage capacitor 358 before activating the motor 22.

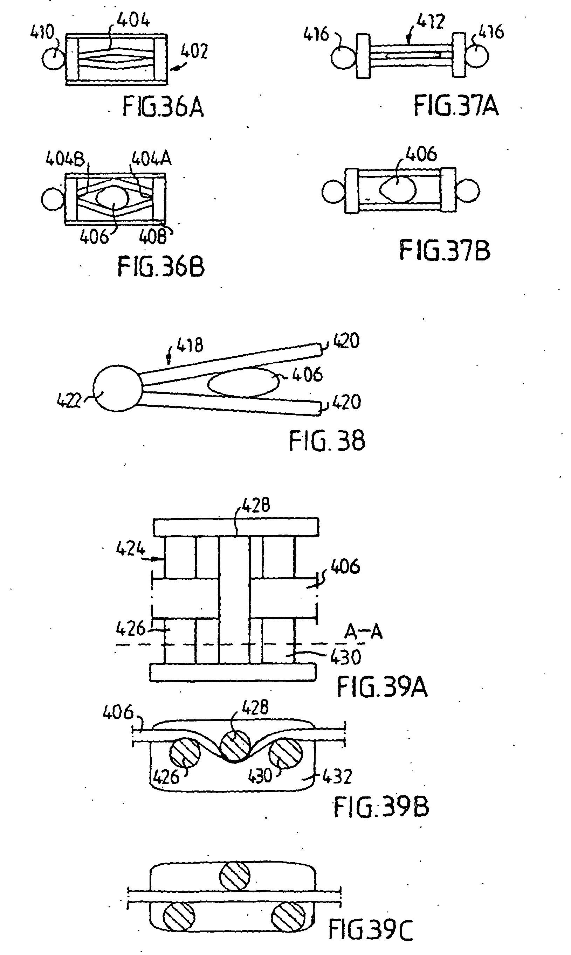

FIGURES 36A and 36B show an embodiment of the apparatus of the invention comprising a restriction device 402 having an elongated flexible restriction member 404, such as a belt, a cord or the like. The flexible member 404 extends in a loop around the tissue, suitably an exit vein from the penis. (Alternatively, the flexible member 404 may comprise two separate parts on opposite sides of the vein.) One portion 404A of member 404 is attached to a frame 408 and another portion 404B of member 404 opposite portion 404A in the loop of the flexible member 404 is connected to an adjustment device 410, which is fixed to the frame 408. The adjustment device 410 pulls the flexible member 404 in the direction from portion 404A to squeeze the vein between two opposite lengths of the flexible member 404 to thereby restrict the blood flow in the vein 406, see FIGURE 36A, and releases the vein from the flexible member 404 to thereby increase the blood flow in the vein 406, see FIGURE 36B.

FIGURES 37A and 37B show an embodiment of the apparatus of the invention comprising a restriction device 412 having two rigid plate or bar elements 414 on opposite sides of the vein 406. An adjustment device 416 moves the rigid elements 412 in parallel towards each other to squeeze the vein 406 between the rigid elements 412 to thereby restrict the blood flow in the vein 406, see FIGURE 37A, and moves the rigid elements 412 away from each other to release the vein 406, see FIGURE 37B.

FIGURE 38 shows an embodiment of the apparatus of the invention comprising a restriction device 418 having two rigid articulated clamping elements 420 positioned on opposite sides of the vein 406. An adjustment device 422 turns the clamping elements 420 toward each other to clamp the vein 406 between the clamping elements 420 to thereby restrict the blood flow in the vein 406, and turns the clamping elements 420 away from each other to release the vein 406 from the clamping elements 420 to thereby increase the blood flow in the vein 406.

FIGURES 39A, 39B and' 39C show an embodiment of the apparatus of the invention comprising a restriction device 424 having three bending members in the form of cylindrical rollers 426, 428 and 430 displaced relative one another in a row along the vein 406 and positioned alternately on opposite sides of the vein 406. (Alternatively, each roller 426, 428 and 430 may take the shape of an hour-glass.) An adjustment device 432 moves the two outer rollers 426,430 laterally against the vein 406 in one direction and the intermediate roller 428 against the vein 406 in the opposite direction to bend the vein to thereby restrict the blood flow in the vein 406, see FIGURE 39B. To increase or restore the blood flow in the vein 406, the adjustment device 432 moves the rollers 426-430 away from the vein 406 to release the vein from the rollers 426-430, see FIGURE 39C..

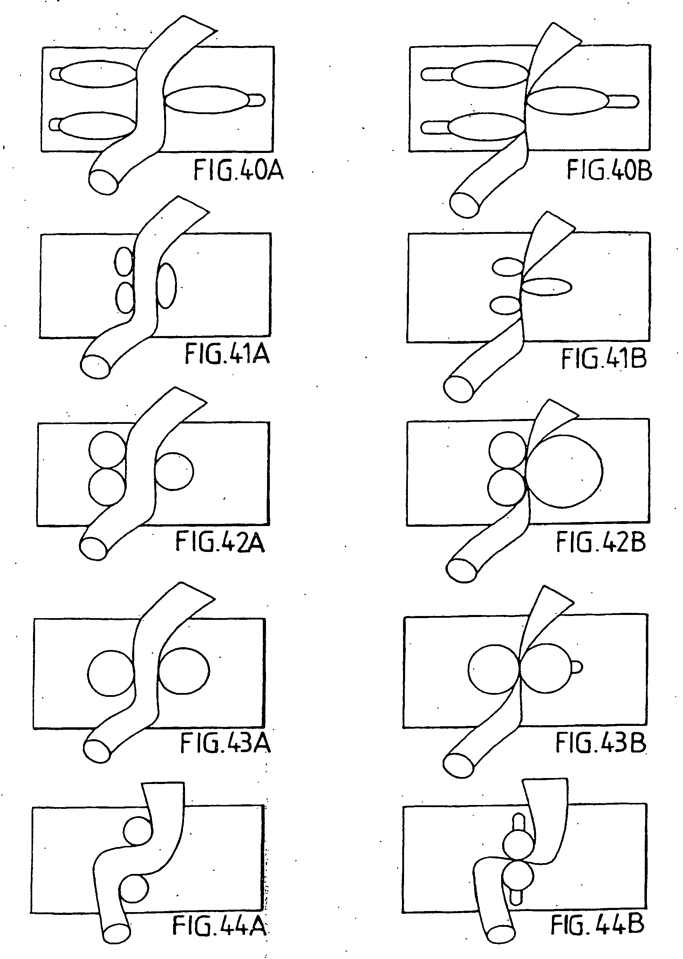

FIGURES 40A through 44B schematically illustrates modifications of the above embodiment according to FIGURES 39A-39C. Thus, FIGURES 40A and 40B show an embodiment similar to that of FIGURES 39A-39C except that the bending members are oval and not rotatable. FIGURES 41A and 41B show an embodiment similar to that of FIGURES 40A and 40B except that the oval bending members are rotatable to release the vein, see FIGURE 41A, and squeeze the vein, see FIGURE 41B. FIGURES 42A and 42B show an embodiment similar to that of FIGURES 39A-39C except that the intermediate roller has a changeable diameter to release the vein, see FIGURE 42A, and squeeze the vein, see FIGURE 42B. FIGURES 43A and 43B show an embodiment similar to that of FIGURES 37A-37C except that the rigid elements are replaced by two cylindrical rollers positioned on opposite sides of the vein. Finally, FIGURES 44A. and 44B. show an embodiment substantially similar to that of FIGURES 43A and 43B except that the restriction device is curved to form an S-shaped curvature of the vein.

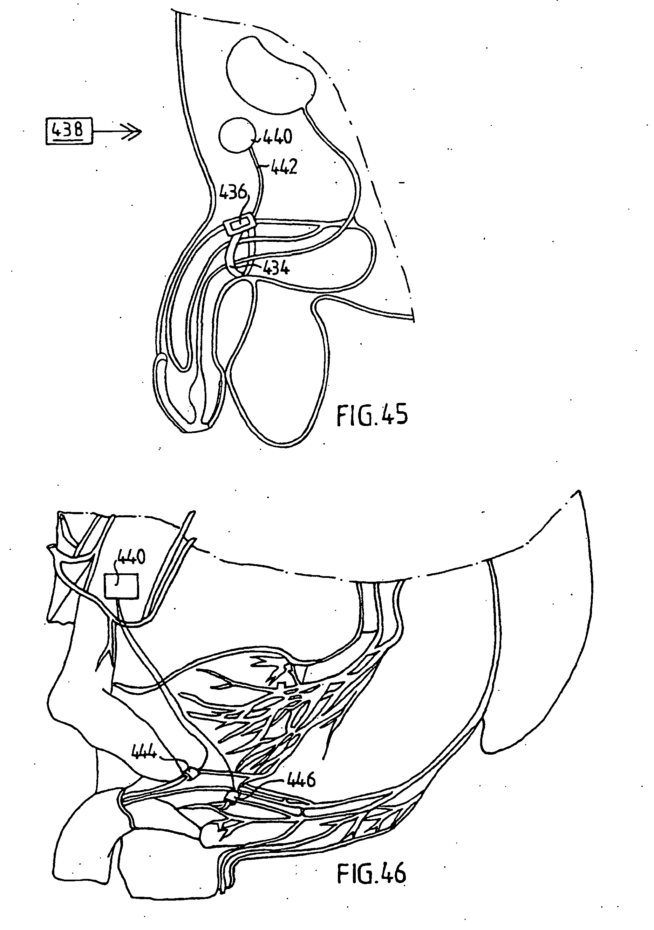

FIGURE 45 schematically illustrates how any of the above-described embodiments of the male sexual impotence treatment apparatus of the invention may be implanted in a patient. Thus, the apparatus comprises an adjustable restriction device 434 extending around the corpus cavernosum of the patient and a motor operated adjustment device 436 for mechanically adjusting the restriction device 434 to squeeze the corpus cavernosum to thereby restrict the blood flow leaving the penis. The motor, not shown, is integrated in the adjustment device 436 and is reversible to operate the adjustment device 436 to release the corpus cavernosum from the restriction device 434. A wireless remote control of the apparatus comprises an external signal transmitter 438 incorporated in a portable remote-control case and an implanted signal receiver 440, which comprises a control unit for controlling the adjustment device 436 in response to a control signal, for example an electromagnetic wave signal, from the transmitter 438. The signal receiver 440 further comprises an energiser unit which transfers energy from the control signal transmitted by the transmitter 438 into electric energy for energy consuming implanted components of the apparatus, such as the motor for operating the adjustment device 436. The electric energy is conducted via an implanted conductor 442 from the signal receiver 440 to the motor. When the patient wishes to achieve erection, he readily uses the portable remote-control case to activate the implanted adjustment device 436 to temporarily adjust the implanted restriction device 434 to restrict the blood flow leaving his penis.

FIGURE 46 shows an embodiment which is identical to the embodiment of FIGURE 45, except that the restriction device comprises two restriction members 444 and 446 extending around respective exit veins from the penis. A motor operated adjustment device, not shown, is incorporated in each restriction member 444, 446.

There are a number of other conceivable alternative embodiments of the invention that give the same result as the above-described embodiments. For example, the microprocessor of the external and implanted, respectively, control unit may be replaced by discrete components. The power amplifier of the external control unit may be omitted if the signals generated by the signal generator are strong enough. Therefore the invention is to be accorded the broadest interpretation of the appended claims to encompass all equivalent structures and assemblies.

## Claims

1. A male sexual impotence treatment apparatus, comprising an adjustable restriction device (2;48;60,62;88;110;122;126,128;130;434;444,446) implantable in a male impotent patient for directly engaging a portion of the normal penile tissue or the prolongation thereof of the patient, an operable adjustment device (12;52;66;90,92;104;110;436) implantable in the patient and adapted to mechanically adjust the restriction device to temporarily contract said portion of the normal penile tissue or the prolongation thereof to restrict the blood flow leaving the penis, when the patient desires to achieve erection, and an implantable operation device for operating the adjustment device, wherein the operation device comprises hydraulic means (54) for operating the adjustment device (52) and that the apparatus further comprises an implantable reservoir (204;210;216) containing a predetermined amount of fluid for supplying the hydraulic means with fluid, **characterised in that** the operation device is powered to operate the adjustment device.

2. An apparatus according to claim 1, wherein the restriction device is operable to contract said portion of the patient's penile tissue or the prolongation thereof, when the patient desires to achieve erection, such that the penile arterial blood flow is at least substantially unrestricted, whereas the penile venous blood flow is substantially restricted.

3. An apparatus according to claim 1 or 2, wherein the restriction device is designed for engagement with both of the corpora cavernosa or crura of the penis or the prolongations thereof as a single unit, or for engagement with each of the two corpora cavernosa or crura or the prolongation thereof as two separate units.

4. An apparatus according to claim 1 or 2, wherein said portion of the penile tissue comprises one or more of the exit veins from the penis.

5. An apparatus according to any of claims 1-4, wherein the adjustment device (12;52;66;90,92;104;110;436) is adapted to adjust the restriction device in a non-magnetic or non-thermal manner.

6. An apparatus according to any of claims 1-6, wherein the restriction device is non-inflatable.

7. An apparatus according to any of claims 1-6, wherein the restriction device comprises an element to be placed on one side of said portion of the penile tissue, and the adjustment device is adapted to squeeze said portion of the penile tissue between the element and the human bone or tissue to decrease the blood flow leaving the penis.

8. An apparatus according to any one of claims 1-6, wherein the restriction device comprises at least one elongated restriction member (2;48;60,62;88) and forming means (10;94;106;108;118;132) for forming the restriction member into at least a substantially closed loop around said portion of the penile tissue, the loop defining a restriction opening, whereby the adjustment device is adapted to adjust the restriction member in the loop to change the size of the restriction opening.

9. An apparatus according to claim 8, wherein the restriction device comprises several elongated restriction members (2;48;60,62;88) to be formed into at least substantially closed loops around the penile tissue.

10. An apparatus according to claim 8 or 9, wherein, the adjustment device (12;52;66;90,92) is adapted to adjust the longitudinal extension of the elongated restriction member in said loop to change the size of the restriction opening.

11. An apparatus according to claim 10, wherein the restriction member (2;48) comprises a main portion and two elongated end portions (4,6), and the adjustment device (12;52;66;90,92) is adapted to establish longitudinal relative displacement between the end portions of the restriction member, such that the size of the restriction opening (3) is adjusted.

12. An apparatus according to claim 1, wherein the adjustment device (12;52;66;90,92) comprises a movement transferring member (14;40;78,90;80,92) in engagement with at least one of the end portions (4,6) of the restriction member and operable to displace said one end portion relative to the other end portion of the restriction member.

13. An apparatus according to claim 12, further comprising a motor (22), which is fixed relative to the main portion of the restriction member (2) and has a rotating drive shaft (32) operatively connected to the movement transferring member (14).

14. An apparatus according to claim 13, wherein the motor is positioned relative to the elongated restriction member such that the drive shaft extends in parallel with a chord in said loop of the restriction member.

15. An apparatus according to claim 10, wherein the elongated restriction member (48,50) is longitudinally resilient and the adjustment device comprises a contraction means (52) adapted to longitudinally contract the resilient restriction member.

16. An apparatus according to claim 15, wherein the elongated restriction member comprises a substantially non-resilient main portion (48) and an end portion forming an elongated helical spring (50), which is contractible by the contraction means (52).

17. An apparatus according to claim 16, wherein the contraction means comprises an elongated flexible pulling member (52) connected to the main portion (48) of the restriction member and extending through the helical spring (50) to contract the helical spring against an arresting member (56), which is fixed relative to the main portion of the restriction member.

18. An apparatus according to claim 8, wherein the restriction member comprises an elongated helical spring (60) having a free end, and a body (64) to which said spring is nonrotatably secured at its opposite end, the adjustment device (66) being adapted to rotate the helical spring in one direction to enlarge the coils of the helical spring to longitudinally contract the elongated helical spring and to rotate the helical spring in the opposite direction to reduce the size of the coils of the helical spring to longitudinally extend the helical spring.

19. An apparatus according to claim 18, wherein the restriction member comprises a further elongated helical spring (62) having a free end and nonrotatably secured to the body (64) at its opposite end, and the adjustment device comprises a drive shaft (66) having two opposite end portions connected to the helical springs, respectively, at their free ends, the helical coils forming left and right hand helices, respectively.

20. An apparatus according to claim 19, wherein the restriction member comprises a further elongated helical spring (62) having a free end and nonrotatably secured to the body (64) at its opposite end, and the adjustment device comprises a gearing (70) having an input shaft (78) and two opposite aligned output shafts (74,76) connected to the helical springs (60,62), respectively, at their free ends, the input shaft being connected to the output shafts such that the output shafts rotate in the opposite directions upon rotation of the input shaft, the helical coils forming the same helices.

21. An apparatus according to claim 8, wherein the restriction member (88;110;122) forms a radial innermost circumferential confinement surface in said loop of the restriction member, and the adjustment device (104;112) is adapted to mechanically adjust the restriction member such that at least a portion of the confinement surface is substantially radially displaced in said loop.

22. An apparatus according to claim 21, wherein the adjustment device comprises an elongated voltage responsive element (104) forming part of the confinement surface and capable of bending into a bow in response to a voltage applied across the element, the radius of curvature of said bow being adjustable by changing the level of the voltage.

23. An apparatus according to claim 21, wherein the restriction member comprises an elastic annular element (110) forming the confinement surface, and the adjustment device (112) is adapted to change the diameter of the elastic annular element.

24. An apparatus according to claim 21, wherein the forming means comprises a substantially rigid outer annular element (118), and the restriction member comprises an elongated helical spring (60) extending internally along the outer annular element and contacting the latter, the helical spring forming part of the circumferential confinement surface and having a free end, and a body to which the helical spring is nonrotatably secured at its opposite end, the adjustment device being adapted to rotate the helical spring in one direction to enlarge the coils of the helical spring to contract the circumferential confinement surface and to rotate the helical spring in the opposite direction to reduce the size of the coils of the helical spring to expand the circumferential confinement surface.

25. An apparatus according to claim 21, wherein the forming means comprises a substantially rigid outer annular element (118), and the restriction member comprises a first (60) and a second (62) elongated helical spring extending internally along the outer annular element and contacting the latter, the helical springs forming part of the circumferential confinement surface, the first and the second spring, respectively, having a free end, and a body to which the first and the second spring, respectively, is nonrotatably secured at its opposite end, the adjustment device being adapted to rotate the first and the second spring, respectively, in one direction to enlarge the coils of the spring to contract the circumferential confinement surface and to rotate the first and the second spring, respectively, in the opposite direction to reduce the size of the coils of the spring to expand the circumferential confinement surface.

26. An apparatus according to claim 8, wherein the restriction member comprises at least two separate elements, at least one of which is pivoted such that it is turnable in a plane in which said loop of the restriction member extends, and the adjustment device is adapted to turn said pivoted element to change the size of said restriction opening.

27. An apparatus according to any one of claims 1-6, wherein the restriction device comprises at least two frame elements (126 and 128), which are foldable towards each other by the adjustment device.

28. An apparatus according to claim 27, wherein the foldable frame elements comprise two substantially semi-circular frame elements (126 and 128), which are hinged together such that the semicircular elements are swingable relative to each other from a fully open state in which they substantially form a circle to a fully folded state in which they form a semicircle.

29. An apparatus according to claim 8, wherein the elongated restriction member (130) is elastic and varies in thickness as seen in a cross-section thereof, and the adjustment device is adapted to turn the restriction member around the longitudinal extension thereof.

30. An apparatus according to claim 8, wherein the forming means comprises a spring material forming the elongated restriction member into the loop, such that the restriction opening has a predetermined size, and the adjustment device is adapted to adjust the restriction member against the spring action of the spring material.

31. An apparatus according to claim 30, wherein the spring material is integrated in the restriction member.

32. An apparatus according to claim 8, wherein the forming means (10;94;106;108;118;132) is adapted to form the restriction member (2;48;60,62;88;110;122;126,128;130) into a loop having a predetermined size or a size selected from several predetermined sizes.

33. An apparatus according to claim 8, wherein the adjustment device (12;52;66;90;104;110) is adapted to change the size of the restriction opening (3) such that the outer circumferential confinement surface of the restriction member (2;48;60,62;88;110;122;126,128;130) is changed.

34. An apparatus according to claim 8, wherein the adjustment device (12;52;66;90;104;110;434) is adapted to change the size of the restriction opening (3) such that the outer circumferential confinement surface of the restriction member (2;48;60,62;88;110;122;126,128;130:436) is unchanged.

35. An apparatus according to claim 8, wherein the elongated restriction member (404) is flexible, and the adjustment device (410) is adapted to pull a first portion (404A) of the flexible restriction member from a second portion (404B) of the flexible restriction member opposite the first portion in the loop to squeeze said portion of the penile tissue (406) between two opposite lengths of the elongated flexible restriction member to restrict the blood flow leaving the penis, and to release said portion of the penile tissue (406) from the flexible restriction member to increase the blood flow leaving the penis.

36. An apparatus according to any one of claims 1-6, wherein the restriction device (412) comprises at least two elements (414) to be placed on different sides of said portion of the penile tissue (406), and the adjustment device is adapted to squeeze said portion of the penile tissue (406) between the elements to restrict the blood flow leaving the penis, and to release said portion of the penile tissue (406) from the elements to increase the blood flow leaving the penis.

37. An apparatus according to any one of claims 1-6 wherein the restriction device (418) comprises at least two articulated clamping elements (420) to be positioned on opposite or different sides of said portion of the penile tissue (406), and the adjustment device (422) is adapted to turn the clamping elements toward each other to clamp said portion of the penile tissue (406) between the clamping elements to restrict the blood flow leaving the penis, and to turn the clamping elements away from each other to release said portion of the penile tissue (406) from the clamping elements to increase the blood flow leaving the penis.

38. An apparatus according to any one of claims 1-6, wherein the restriction device is adapted to bend a portion of said portion of the penile tissue (406).

39. An apparatus according to claim 38, wherein the restriction device (424) comprises at least two bending members (426-430) to be positioned on opposite or different sides of said portion of the penile tissue (406) and to be displaced relative to each other along the penile tissue, and the adjustment device (432) is adapted to move the bending members against said portion of the penile tissue (406) to bend it to restrict the blood flow leaving the penis, and to move the bending members away from said portion of the penile tissue (406) to release it from the bending members to increase the blood flow leaving the penis.

40. An apparatus according to claim 39, wherein the bending members comprise rollers.

41. An apparatus according to any one of claims 1-6, wherein the restriction device is adapted to rotate a portion of the penile tissue.

42. An apparatus according to claim 1, wherein the operation device comprises a servo means.

43. An apparatus according to claim 1, wherein the reservoir (210) defines a chamber for the predetermined amount of fluid and the hydraulic means (202) of the operation device is adapted to change the volume of the chamber.

44. An apparatus according to claim 43, wherein the hydraulic means comprises a pump (212) adapted to pump fluid between the reservoir (204) and the adjustment device (202).

45. An apparatus according to claim 43 or 44, wherein the hydraulic means is devoid of any non-return valve.

46. An apparatus according to any one of claims 43-45, wherein the hydraulic means comprises a reverse servo (210,214;276-286).

47. An apparatus according to claim 46, wherein the reverse servo is manually operated.

48. An apparatus according to claim 46, wherein the reverse servo is powered.

49. An apparatus according to any of claims 46-48, wherein the hydraulic means comprises first and second wall portions of the reservoir (204), and the reverse servo is adapted to provide relative displacement between the first and second wall portions of the reservoir.

50. An apparatus according to any one of the preceding claims, further comprising a wireless remote control (22,326,332-344) for non-invasively controlling the adjustment device.

51. An apparatus according to claim 50, wherein the remote control (22,326,332-344) comprises a separate signal transmitter and/or receiver (332,336) and an implantable signal receiver and/or transmitter (334,338), for transmitting and/or receiving a control signal.

52. An apparatus according to claim 51, wherein the signal receiver (334,338) comprises a control unit (338) adapted to control the adjustment device (12;52;66;90,92;104;110) in response to the control signal.

53. An apparatus according to claim 52, further comprising an implantable energiser unit (336) for providing energy to energy consuming components of the apparatus to be implanted in the patient.

54. An apparatus according to any of claims 50-53, further comprising an implantable motor (22) for operating the adjustment device (12;52;66;90,92;104;110).

55. An apparatus according to claims 53 and 54, wherein the control unit (338) is adapted to control the energiser unit (336) to power the motor (22) with energy in response to the control signal.

56. An apparatus according to claim 54 or 55, wherein the motor (22) is an electric motor.

57. An apparatus according to claim 53, wherein the energiser unit (326) is adapted to transform energy from the control signal, as it is transmitted to the signal receiver (334,338), into electric energy.

58. An apparatus according to claim 53 or 57, wherein the energiser unit (26) is adapted to transform energy from the control signal into a direct or alternating current.

59. An apparatus according to claim 58, further comprising an implantable electric motor (22) for operating the adjustment device (12;52;66;90,92;104;110), wherein the energiser unit (326) comprises a rechargeable electric power supply (58) for storing the electric energy and the control unit (338) is adapted to power the electric motor (22) with energy from the rechargeable electric power supply in response to the control signal.

60. An apparatus according to claim 53, wherein the energiser unit (326) comprises a battery, an electrically operable switch adapted to connect the battery to the signal receiver in (334,338) an "on" mode when the switch is powered and to keep the battery disconnected from the signal receiver in a "standby" mode when the switch is not powered, and a rechargeable electric power supply for powering the switch.

61. An apparatus according to claim 60, wherein the control unit (338) is adapted to power the electric motor (22) with energy from the battery in response to a control signal received from the signal transmitter (332,336), when the switch is in its "on" mode.

62. An apparatus according to claim 53, further comprising an external energy transmitter for transmitting wireless energy, wherein the energiser unit comprises a battery and a switch operable by the wireless energy transmitted by the external transmitter, for connecting the battery to the signal receiver in an "on" mode when the switch is powered by the wireless energy and to keep the battery disconnected from the signal receiver in a "standby" mode when the switch is not powered.

63. An apparatus according to claim 1, further comprising an implantable energiser unit (336) for providing energy to energy consuming components of the apparatus to be implanted in the patient.

64. An apparatus according to claim 63, further comprising an external energy transmitter for transmitting wireless energy, wherein the energiser unit is adapted to transform the wireless energy into electric energy.

65. An apparatus according to claim 64, further comprising an implantable electric motor (22) for operating the adjustment device (12;52;66;90,92;104;110), wherein the energiser unit (326) is adapted to power the electric motor (22) with the electric energy transformed from the wireless energy.

66. An apparatus according to claim 63, further comprising an external energy transmitter for transmitting wireless energy, wherein the energiser unit comprises a battery and a switch operable by the wireless energy transmitted by the external transmitter, for connecting the battery to the implantable energy consuming components of the apparatus in an "on" mode when the switch is powered by the wireless energy and to keep the battery disconnected from the energy consuming components in a "standby" mode when the switch is not powered.

67. An apparatus according to claim 62 or 65, wherein the external energy transmitter is adapted to directly power the switch with the wireless energy to switch into the "on" mode.

68. An apparatus according to claim 50, wherein the remote control (22,326,332-344) is capable of obtaining information from implantable components of the apparatus and of commanding the adjustment device (12;52;66;90;104;110) to adjust the restriction device (2;48;60,62;88;110;122;126, 128;130;434) in response to obtained information.

69. An apparatus according to claim 1, further comprising implantable electrical components including at least one voltage level guard.

70. An apparatus according to claim 1, further comprising implantable electrical components including a single voltage level guard.

71. An apparatus according to claim 69 or 70, wherein the electrical components are devoid of any current detector and/or charge level detector.

72. An apparatus according to any of claims 69-71, further comprising an implantable capacitor or accumulator, wherein the charge or discharge of the capacitor or accumulator is controlled by use of the voltage level guard.

73. An apparatus according to claim 1, further comprising an energy transfer means (22,326,332-344) for wireless transfer of energy from outside the patient's body to the adjustment device and/or other energy consuming implantable components of the apparatus.

74. An apparatus according to claim 73, wherein the energy transfer means is adapted to intermittently transfer the energy in the form of a train of energy pulses for direct use in connection with the energising of the energy consuming components of the apparatus.

75. An apparatus according to claim 74, wherein the energy transfer means is adapted to transfer electric energy, and further comprising an implantable capacitor for producing the train of energy pulses.

76. An apparatus according to claim 72 or 75, wherein the capacitor has a capacity less than 0,1 µF.

77. An apparatus according to claim 73, further comprising an implantable motor (22) or pump for operating the adjustment device (12;52;66;90,92;104;110), wherein the energy transfer means is adapted to directly power the motor or pump with transferred energy.

78. An apparatus according to claim 1, wherein the powered operation device comprises an implantable motor or pump for operating the adjustment device, wherein the energy transmission device is adapted to transmit wireless energy in the form of an magnetic field or electromagnetic waves for direct power of the motor or pump, as the wireless energy is being transmitted.

79. An apparatus according to claim 78, wherein the pump is not a plunger type of pump.

80. An apparatus according to claim 73, wherein the energy transfer means is adapted to transfer wireless energy in the form of electromagnetic waves excluding radio waves.

81. An apparatus according to claims 73 or 74, wherein the energy transferred by the energy transfer means comprises an electric field or a magnetic field.

82. An apparatus according to claims 73 or 74, wherein the energy transferred by the energy transfer means comprises a signal.

83. An apparatus according to claim 51 or 82, wherein the signal comprises analog or digital pulses.

84. An apparatus according to claim 83, wherein the analog or digital pulses comprise a magnetic field or an electric field.

85. An apparatus according to claim 51, 81 or 82, wherein the signal comprises a wave signal.

86. An apparatus according to claim 85, wherein the wave signal comprises an electromagnetic wave signal, a sound wave signal or a carrier wave signal.

87. An apparatus according to any one of the preceding claims, further comprising a pressure sensor for directly or indirectly sensing the pressure against the restriction device.

88. An apparatus according to claim 87, wherein the restriction device is controlled in response to signals from the pressure sensor.

89. An apparatus according to claim 1, further comprising an implantable energy transforming device for transforming wireless energy directly or indirectly into energy different than the wireless energy for operation of the restriction device.

90. An apparatus according to claim 89, wherein the energy transforming device transforms the wireless energy into kinetic energy for operation of the restriction device.

91. An apparatus according to claim 89, wherein the energy transforming device transforms the wireless energy in the form of sound waves into electric energy for operation of the restriction device.

92. An apparatus according to any one of the preceding claims, further comprising an implantable reversing device, wherein the restriction device is capable of performing a reversible function and the reversing device reverses the reversible function.

93. An apparatus according to any one of the preceding claims, further comprising an implantable accumulator or battery and means for controlling the accumulator or battery from outside the patient's body to supply energy to the adjustment device and/or other implantable energy consuming components of the apparatus.

94. An apparatus according to claim 1, wherein the adjustment device is adapted to adjust the restriction device in a non-manual manner.

95. An apparatus according to any of the preceding claims, wherein the restriction device is designed for implantation in the base of the patient's penis or its prolongation.

96. An apparatus according to claim 91, wherein the energy transforming device transforms the wireless energy in the form of sound waves directly into electric energy.

97. An apparatus according to claim 73, 91 or 96, wherein the energy transforming device comprises a capacitor.

98. An apparatus according to claim 97, wherein the capacitor is adapted to produce electric pulses from the transformed electric energy.

99. An apparatus according to claim 48, wherein the reverse servo comprises a motor, preferably an electric motor.

100. An apparatus according to claim 99, wherein the motor is reversible.

101. An apparatus according to claim 86, wherein the carrier signal is frequency, amplitude or frequency and amplitude modulated.

102. An apparatus according to claim 86, wherein the control signal comprises a wave signal comprising one of a sound wave signal including an ultrasound wave signal, an electromagnetic wave signal including an infrared light signal, a visible light signal, an ultra violet light signal and a laser light signal, a micro wave signal, a radio wave signal, an x-ray radiation signal, and a gamma radiation signal.

103. An apparatus according to claim 1, wherein the hydraulic means of the operation device comprises a fluid conduit, the reservoir forming part of the conduit.

104. An apparatus according to claim 103, wherein the hydraulic means including the conduit are devoid of any non-return valve.

105. An apparatus according to claim 73, wherein the energy transfer means is adapted to transfer magnetic energy, non-magnetic energy, electromagnetic energy, non-electromagnetic energy, kinetic energy, non-kinetic energy, sonic energy, non-sonic energy, thermal energy or non-thermal energy.

106. An apparatus according to claim 4, wherein the restriction device is adapted to control the cross-sectional area of a blood flow passageway formed by the patient's penile exit veins.

107. An apparatus according to claim 106, wherein the restriction device is operable to open and close the blood flow passageway.

108. An apparatus according to claim 107, wherein the restriction device is adapted to steplessly control the cross-sectional area of the blood flow passageway.

## Patentansprüche

1. Vorrichtung zur Behandlung männlicher sexueller Impotenz, aufweisend eine einstellbare Begrenzungsvorrichtung (2;48;60,62;88;110;122;126;128;130;434;444;446), die in einem männlichen impotenten Patienten für den direkten Eingriff eines Abschnitts des normalen Penisgewebes oder dessen Verlängerung des Patienten implantierbar ist, eine betätigbare Einstellvorrichtung (12;52;66;90;92;104;110;436), die in dem Patienten implantierbar ist und geeignet ist, die Begrenzungsvorrichtung mechanisch einzustellen, um den Abschnitt des normalen Penisgewebes oder dessen Verlängerung temporär zusammenzuziehen, um den aus dem Penis austretenden Blutfluss zu begrenzen, wenn der Patient wünscht, eine Erektion zu erreichen, und eine implantierbare Betätigungsvorrichtung zum Betätigen der Einstellvorrichtung, wobei die Betätigungsvorrichtung Hydraulikmittel (54) zum Betätigen der Einstellvorrichtung (52) aufweist, und die Vorrichtung ferner einen implantierbaren Behälter (204;210;216) aufweist, der eine vorbestimmte Menge von Fluid zum Versorgen des Hydraulikmittels mit Fluid, **dadurch gekennzeichnet, dass** die Betätigungsvorrichtung angetrieben wird, um die Einstellvorrichtung zu betätigen.

2. Vorrichtung nach Anspruch 1, wobei die Begrenzungsvorrichtung betätigbar ist, um den Abschnitt des Penisgewebes oder dessen Verlängerung des Patienten zusammenzuziehen, wenn der Patient wünscht, eine Erektion zu erreichen, so dass der Penisarterienblutfluss wenigstens im Wesentlichen unbegrenzt ist, wohingegen der Penisvenenblutfluss im Wesentlichen begrenzt ist.

3. Vorrichtung nach Anspruch 1 oder 2, wobei die Begrenzungsvorrichtung für den Eingriff mit den beiden Schwellkörpern oder Schenkeln des Penis oder dessen Verlängerung als eine einzige Einheit oder für den Eingriff mit jedem der beiden Schwellkörper oder Schenkel oder dessen Verlängerung als zwei separate Einheiten gestaltet ist.

4. Vorrichtung nach Anspruch 1 oder 2, wobei der Abschnitt des Penisgewebes eine oder mehrere der aus dem Penis austretenden Venen umfasst.

5. Vorrichtung nach einem der Ansprüche 1-4, wobei die Einstellvorrichtung (12;52;66;90;92;104;110;436) geeignet ist, die Begrenzungsvorrichtung in einer nichtmagnetischen oder nichtthermischen Weise einzustellen.

6. Vorrichtung nach einem der Ansprüche 1-5, wobei die Begrenzungsvorrichtung nichtaufblasbar ist.

7. Vorrichtung nach einem der Ansprüche 1-6, wobei die Begrenzungsvorrichtung ein Element aufweist, das an einer Seite des Abschnitts des Penisgewebes zu platzieren ist, und die Einstellvorrichtung geeignet ist, den Abschnitt des Penisgewebes zwischen dem Element und dem menschlichen Knochen oder Gewebe zu quetschen, um den aus dem Penis austretenden Blutfluss zu verringern.

8. Vorrichtung nach einem der Ansprüche 1-6, wobei die Begrenzungsvorrichtung wenigstens ein verlängertes Begrenzungselement (2;48;60,62;88) und Formungsmittel (10;94;106;108;118;132) zum Formen des Begrenzungselements zu wenigstens einer im Wesentlichen geschlossenen Schlinge um den Abschnitt des Penisgewebes herum aufweist, wobei die Schlinge eine Begrenzungsöffnung definiert, wodurch die Einstellvorrichtung geeignet ist, das Begrenzungselement in der Schlinge einzustellen, um die Größe der Begrenzungsöffnung zu verändern.

9. Vorrichtung nach Anspruch 8, wobei die Begrenzungsvorrichtung mehrere verlängerte Begrenzungselemente (2;48;60,62;88) aufweist, die zu wenigstens im Wesentlichen geschlossenen Schlingen um das Penisgewebe herum zu formen sind.

10. Vorrichtung nach Anspruch 8 oder 9, wobei die Einstellvorrichtung (12;52;66;90,92) geeignet ist, die Längserstreckung des verlängerten Begrenzungselements in der Schlinge einzustellen, um die Größe der Begrenzungsöffnung zu verändern.

11. Vorrichtung nach Anspruch 10, wobei das Begrenzungselement (2;48) einen Hauptabschnitt und zwei verlängerte Endabschnitte (4,6) aufweist, und die Einstellvorrichtung (12;52;66;90,92) geeignet ist, eine relative Längsverschiebung zwischen den Endabschnitten des Begrenzungselements derart zu bilden, dass die Größe der Begrenzungsöffnung (3) eingestellt wird.

12. Vorrichtung nach Anspruch 1, wobei die Einstellvorrichtung (12;52;66;90,92) ein Bewegungsübertragungselement (14;40;78;90;80,92) in Eingriff mit wenigstens einem der Endabschnitte (4,6) des Begrenzungselements aufweist und betätigbar ist, um den einen Endabschnitt relativ zu dem anderen Endabschnitt des Begrenzungselements zu verschieben.

13. Vorrichtung nach Anspruch 12, ferner aufweisend einen Motor (22), welcher relativ zu dem Hauptabschnitt des Begrenzungselements (2) befestigt ist und eine drehbare Antriebswelle (32) aufweist, die mit dem Bewegungsübertragungselement (14) wirksam verbunden ist.

14. Vorrichtung nach Anspruch 13, wobei der Motor relativ zu dem verlängerten Begrenzungselement derart positioniert ist, dass sich die Antriebswelle parallel zu einem Strang in der Schlinge des Begrenzungselements erstreckt.

15. Vorrichtung nach Anspruch 10, wobei das verlängerte Begrenzungselement (48,50) längselastisch ist und die Einstellvorrichtung ein Kontraktionsmittel (52) aufweist, das geeignet ist, das elastische Begrenzungsmittel längs zusammenzuziehen.

16. Vorrichtung nach Anspruch 15, wobei das verlängerte Begrenzungselement einen im Wesentlichen nichtelastischen Hauptabschnitt (48) und einen Endabschnitt aufweist, der eine verlängerte Schraubenfeder (50) bildet, welche durch das Kontraktionsmittel (52) zusammenziehbar ist.

17. Vorrichtung nach Anspruch 16, wobei das Kontraktionsmittel ein verlängertes flexibles Zugmittel (52) aufweist, das mit dem Hauptabschnitt (48) des Begrenzungselements verbunden ist und sich durch die Schraubenfeder (50) hindurch erstreckt, um die Schraubenfeder gegen ein Anschlagelement (56) zusammenzuziehen, welches relativ zu dem Hauptabschnitt des Begrenzungselements befestigt ist.

18. Vorrichtung nach Anspruch 8, wobei das Begrenzungselement eine verlängerte Schraubenfeder (60) mit einem freien Ende und einen Körper (64) aufweist, an welchem die Feder mit ihrem entgegengesetzten Ende nichtdrehbar gesichert ist, wobei die Einstellvorrichtung (66) geeignet ist, die Schraubenfeder in der einen Richtung zu drehen, um die Wicklungen der Schraubenfeder zu erweitern, damit sich die verlängerte Schraubenfeder längs zusammenzieht und damit sich die Schraubenfeder in der entgegengesetzten Richtung dreht, um die Größe der Windungen der Schraubenfeder zu reduzieren, damit sich die Schraubenfeder längs erweitert.

19. Vorrichtung nach Anspruch 18, wobei das Begrenzungselement eine weitere verlängerte Schraubenfeder (62) aufweist, die ein freies Ende hat und mit ihrem entgegengesetzten Ende an dem Körper (64) nichtdrehbar gesichert ist, und die Einstellvorrichtung eine Antriebswelle (66) mit zwei entgegengesetzten Endabschnitten aufweist, die mit den jeweiligen Schraubenfedern an ihren freien Enden verbunden sind, wobei die Schraubenwindungen links- bzw. rechtsgängige Schraubenlinien bilden.

20. Vorrichtung nach Anspruch 19, wobei das Begrenzungselement eine weitere verlängerte Schraubenfeder (62) aufweist, die ein freies Ende hat und mit ihrem entgegengesetzten Ende an dem Körper (64) nichtdrehbar gesichert ist, und die Einstellvorrichtung ein Getriebe (70) mit einer Antriebswelle (72) und zwei entgegengesetzt zueinander ausgerichtete Abtriebswellen (74,76) aufweist, die mit den jeweiligen Schraubenfedern (60,62) an ihren freien Enden verbunden sind, wobei die Antriebswelle mit den Abtriebswellen derart verbunden ist, dass sich die Abtriebswellen bei einer Drehung der Antriebswelle in den entgegengesetzten Richtungen drehen, wobei die Schraubenwindungen dieselben Schraubenlinien bilden.

21. Vorrichtung nach Anspruch 8, wobei das Begrenzungselement (88;110;122) eine radial innerste Umfangsbegrenzungsfläche in der Schlinge des Begrenzungselements bildet, und die Einstellvorrichtung (104;112) geeignet ist, das Begrenzungselement derart mechanisch einzustellen, dass wenigstens ein Abschnitt der Begrenzungsfläche in der Schlinge im Wesentlichen radial verschoben wird.

22. Vorrichtung nach Anspruch 21, wobei die Einstellvorrichtung ein verlängertes spannungsreagierendes Element (104) aufweist, das einen Teil der Begrenzungsfläche bildet und zum Biegen zu einem Bogen in Reaktion auf eine über dem Element ausgeübte Spannung geeignet ist, wobei der Krümmungsradius des Bogens durch Veränderung der Größe der Spannung einstellbar ist.

23. Vorrichtung nach Anspruch 21, wobei das Begrenzungselement ein elastisches Ringelement (110) aufweist, das die Begrenzungsfläche bildet, und die Einstellvorrichtung (112) geeignet ist, den Durchmesser des elastischen Ringelements zu verändern.

24. Vorrichtung nach Anspruch 21, wobei das Formungsmittel ein im Wesentlichen starres Außenringelement (118) aufweist, und das Begrenzungselement eine verlängerte Schraubenfeder (60), die sich innen entlang des Außenringelements erstreckt und das letztere kontaktiert, wobei die Schraubenfeder einen Teil der Umfangsbegrenzungsfläche bildet und ein freies Ende hat, und einen Körper aufweist, an welchem die Schraubenfeder mit ihrem entgegengesetzten Ende nichtdrehbar gesichert ist, wobei die Einstellvorrichtung geeignet ist, die Schraubenfeder in der einen Richtung zu drehen, um die Windungen der Schraubenfeder zu erweitern, damit sich die Umfangsbegrenzungsfläche zusammenzieht, und die Schraubenfeder in der entgegengesetzten Richtung zu drehen, um die Größe der Windungen der Schraubenfeder zu reduzieren, damit die Umfangsbegrenzungsfläche expandiert.

25. Vorrichtung nach Anspruch 21, wobei das Formungsmittel ein im Wesentlichen starres Außenringelement (118) aufweist, und das Begrenzungselement eine erste (60) und eine zweite (62) verlängerte Schraubenfeder aufweist, die sich innen entlang des Außenringelements erstrecken und das letztere kontaktieren, wobei die Schraubenfedern einen Teil der Umfangsbegrenzungsfläche bilden und die erste und die zweite Feder jeweils ein freies Ende haben, und einen Körper aufweist, an welchem die erste bzw. die zweite Feder mit ihrem entgegengesetzten Ende nichtdrehbar gesichert ist, wobei die Einstellvorrichtung geeignet ist, die erste bzw. die zweite Feder in der einen Richtung zu drehen, um die Windungen der Feder zu erweitern, damit sich die Umfangsbegrenzungsfläche zusammenzieht, und die erste bzw. die zweite Feder in der entgegengesetzten Richtung zu drehen, um die Größe der Windungen der Feder zu reduzieren, damit die Umfangsbegrenzungsfläche expandiert.

26. Vorrichtung gemäß Anspruch 8, wobei das Begrenzungselement wenigstens zwei separate Elemente aufweist, von denen wenigstens eines derart geschwenkt wird, dass es in einer Ebene drehbar ist, in welcher sich die Schlinge des Begrenzungselements erstreckt, und die Einstellvorrichtung geeignet ist, das geschwenkte Element zu drehen, um die Größe der Begrenzungsöffnung zu verändern.

27. Vorrichtung nach einem der Ansprüche 1-6, wobei die Begrenzungsvorrichtung wenigstens zwei Rahmenelemente (126 und 128) aufweist, welche durch die Einstellvorrichtung in Richtung aufeinander zu faltbar sind.

28. Vorrichtung nach Anspruch 27, wobei die faltbaren Rahmenelemente zwei im Wesentlichen halbkreisförmige Rahmenelemente (126 und 128) aufweisen, welche derart aneinandergelenkt sind, dass die halbkreisförmigen Elemente relativ zueinander aus einem vollständig offenen Zustand, in welchem sie im Wesentlichen einen Kreis bilden, in einen vollständig gefalteten Zustand schwenkbar sind, in welchem sie einen Halbkreis bilden.

29. Vorrichtung nach Anspruch 8, wobei das verlängerte Begrenzungselement (130) elastisch ist und in der Dicke in seinem Querschnitt betrachtet variiert, und die Einstellvorrichtung geeignet ist, das Begrenzungselement um seine Längserstreckung herum zu drehen.

30. Vorrichtung nach Anspruch 8, wobei das Formungsmittel ein Federmaterial aufweist, welches das verlängerte Begrenzungselement zu der Schlinge derart formt, dass die Begrenzungsöffnung eine vorbestimmte Größe hat, und die Einstellvorrichtung geeignet ist, das Begrenzungselement gegen die Federwirkung des Federmaterials einzustellen.

31. Vorrichtung nach Anspruch 30, wobei das Federmaterial in dem Begrenzungselement integriert ist.

32. Vorrichtung nach Anspruch 8, wobei das Formungsmittel (10;94;106;108;118;132) geeignet ist, das Begrenzungselement (2;48;60;62;88;110;122;126;128;130) zu einer Schlinge mit einer vorbestimmten Größe oder einer aus mehreren vorbestimmten Größen ausgewählten Größe zu formen.

33. Vorrichtung nach Anspruch 8, wobei die Einstellvorrichtung (12;52;66;90;104;110) geeignet ist, die Größe der Begrenzungsöffnung (3) derart zu verändern, dass die Außenumfangsbegrenzungsfläche des Begrenzungselements (2;48;60;62;88;110;122;126;128;130) geändert wird.

34. Vorrichtung nach Anspruch 8, wobei die Einstellvorrichtung (12;52;66;90;104;110;434) geeignet ist, die Größe der Begrenzungsöffnung (3) derart zu verändern, dass die Außenumfangsbegrenzungsfläche des Begrenzungselements (2;48;60;62;88;110;122;126;128;130;436) nicht geändert wird.

35. Vorrichtung nach Anspruch 8, wobei das verlängerte Begrenzungselement (404) flexibel ist, und die Einstellvorrichtung (410) geeignet ist, einen ersten Abschnitt (404A) des flexiblen Begrenzungselements von einem zweiten Abschnitt (404B) des flexiblen Begrenzungselements entgegengesetzt zu dem ersten Abschnitt in der Schlinge zu ziehen, um den Abschnitt des Penisgewebes (406) zwischen zwei gegenüberliegenden Abschnitten des verlängerten flexiblen Begrenzungselements zu quetschen, damit der aus dem Penis austretende Blutfluss begrenzt wird, und den Abschnitt des Penisgewebes (406) von dem flexiblen Begrenzungselement freizugeben, damit der aus dem Penis austretende Blutfluss erhöht wird.

36. Vorrichtung nach einem der Ansprüche 1-6, wobei die Begrenzungsvorrichtung (412) wenigstens zwei Elemente (414) aufweist, die an unterschiedlichen Seiten des Abschnitts des Penisgewebes (406) zu platzieren sind, und die Einstellvorrichtung geeignet ist, den Abschnitt des Penisgewebes (406) zwischen den Elementen zu quetschen, um den aus dem Penis austretenden Blutfluss zu begrenzen, und den Abschnitt des Penisgewebes (406) von den Elementen freizugeben, um den aus dem Penis austretenden Blutfluss zu erhöhen.

37. Vorrichtung nach einem der Ansprüche 1-6, wobei die Begrenzungsvorrichtung (418) wenigstens zwei gelenkige Klemmelemente (420) aufweist, die an gegenüberliegenden oder anderen Seiten des Abschnitts des Penisgewebes (406) zu positionieren sind, und die Einstellvorrichtung (422) geeignet ist, die Klemmelemente in Richtung zueinander zu drehen, um den Abschnitt des Penisgewebes (406) zwischen den Klemmelementen zu klemmen, damit der aus dem Penis austretende Blutfluss begrenzt wird, und die Klemmelemente voneinander weg zu drehen, um den Abschnitt des Penisgewebes (406) von den Klemmelementen freizugeben, damit der aus dem Penis austretende Blutfluss erhöht wird.

38. Vorrichtung nach einem der Ansprüche 1-6, wobei die Begrenzungsvorrichtung geeignet ist, einen Teil des Abschnitts des Penisgewebes (406) zu biegen.

39. Vorrichtung nach Anspruch 38, wobei die Begrenzungsvorrichtung (424) wenigstens zwei Biegeelemente (426-430) aufweist, die an gegenüberliegenden oder anderen Seiten des Abschnitts des Penisgewebes (406) zu positionieren sind und relativ zueinander entlang des Penisgewebes zu verschieben sind, und die Einstellvorrichtung (432) geeignet ist, die Biegeelemente gegen den Abschnitt des Penisgewebes (406) zu bewegen, um diesen zu biegen, damit der aus dem Penis austretende Blutfluss begrenzt wird, und die Biegeelemente von dem Abschnitt des Penisgewebes (406) weg zu bewegen, um diesen von den Biegeelementen freizugeben, damit der aus dem Penis austretende Blutfluss erhöht wird.

40. Vorrichtung nach Anspruch 39, wobei die Biegeelemente Rollen aufweisen.

41. Vorrichtung nach einem der Ansprüche 1-6, wobei die Begrenzungsvorrichtung geeignet ist, einen Abschnitt des Penisgewebes zu drehen.

42. Vorrichtung nach Anspruch 1, wobei die Betätigungsvorrichtung ein Servomittel aufweist.

43. Vorrichtung nach Anspruch 1, wobei der Behälter (210) eine Kammer für die vorbestimmte Menge von Fluid definiert, und das Hydraulikmittel (202) der Betätigungsvorrichtung geeignet ist, das Volumen der Kammer zu verändern.

44. Vorrichtung nach Anspruch 43, wobei das Hydraulikmittel eine Pumpe (212) aufweist, die geeignet ist, Fluid zwischen dem Behälter (204) und der Einstellvorrichtung (202) zu pumpen.

45. Vorrichtung nach Anspruch 43 oder 44, wobei das Hydraulikmittel frei von irgendeinem Rückschlagventil ist.

46. Vorrichtung nach einem der Ansprüche 43-45, wobei das Hydraulikmittel einen Umschaltservo (210, 214; 276-286) aufweist.

47. Vorrichtung nach Anspruch 46, wobei der Umschaltservo manuell betätigt wird.

48. Vorrichtung nach Anspruch 46, wobei der Umschaltservo angetrieben wird.

49. Vorrichtung nach einem der Ansprüche 46-48, wobei das Hydraulikmittel erste und zweite Wandabschnitte des Behälters (204) aufweist, und der Umschaltservo geeignet ist, eine relative Verschiebung zwischen den ersten und zweiten Wandabschnitten des Behälters zu schaffen.

50. Vorrichtung nach einem der vorhergehenden Ansprüche, ferner aufweisend eine Funkfernsteuerung (22,326,332-344) zur nichtinvasiven Steuerung der Einstellvorrichtung.

51. Vorrichtung nach Anspruch 50, wobei die Fernsteuerung (22,326,332-344) einen separaten Signal-Sender und/oder Empfänger (332,336) und einen implantierbaren Signal-Empfänger und/oder Sender (334,338) zum Senden und/oder Empfangen eines Steuersignals aufweist.

52. Vorrichtung nach Anspruch 51, wobei der Signalempfänger (334, 338) eine Steuereinrichtung (338) aufweist, die geeignet ist, die Einstellvorrichtung (12;52;66;90;92;104;110) in Reaktion auf das Steuersignal zu steuern.

53. Vorrichtung nach Anspruch 52, ferner aufweisend eine implantierbare Energieversorgungseinrichtung (336) zum Bereitstellen von Energie an Energieverbrauchskomponenten der in dem Patienten zu implantierenden Vorrichtung.

54. Vorrichtung nach einem der Ansprüche 50-53, ferner aufweisend einen implantierbaren Motor (22) zum Betätigen der Einstellvorrichtung (12;52;66;90;92;104;110).

55. Vorrichtung nach den Ansprüchen 53 und 54, wobei die Steuereinrichtung (338) geeignet ist, die Energieversorgungseinrichtung (336) zu steuern, um den Motor (22) in Reaktion auf das Steuersignal mit Energie anzutreiben.

56. Vorrichtung nach Anspruch 54 oder 55, wobei der Motor (22) ein Elektromotor ist.

57. Vorrichtung nach Anspruch 53, wobei die Energieversorgungseinrichtung (326) geeignet ist, Energie von dem Steuersignal, wenn es an den Signalempfänger (334,338) übertragen wird, in elektrische Energie umzuwandeln.

58. Vorrichtung nach Anspruch 53 oder 57, wobei die Energieversorgungseinrichtung (26) geeignet ist, Energie von dem Steuersignal in einen Gleich- oder Wechselstrom umzuwandeln.

59. Vorrichtung nach Anspruch 58, ferner aufweisend einen implantierbaren Elektromotor (22) zum Betätigen der Einstellvorrichtung (12;52;66;90;92;104;110), wobei die Energieversorgungseinrichtung (326) eine wiederaufladbare elektrische Energieversorgung (58) zum Speichern der elektrischen Energie aufweist, und die Steuereinrichtung (338) geeignet ist, den Elektromotor (22) von der wiederaufladbaren elektrischen Energieversorgung in Reaktion auf das Steuersignal mit Energie anzutreiben.

60. Vorrichtung nach Anspruch 53, wobei die Energieversorgungseinrichtung (326) eine Batterie, einen elektrisch betätigbaren Schalter, der geeignet ist, die Batterie mit dem Signalempfänger (334,338) in einem Modus "Ein" zu verbinden, wenn der Schalter eingeschaltet ist, und die Batterie von dem Signalempfänger in einem Modus "Bereitschaft" getrennt zu halten, wenn der Schalter ausgeschaltet ist, und eine wiederaufladbare elektrische Energieversorgung zum Betreiben des Schalters aufweist.

61. Vorrichtung nach Anspruch 60, wobei die Steuereinrichtung (338) geeignet ist, den Elektromotor (22) mit Energie von der Batterie in Reaktion auf ein von dem Signalsender (332,336) empfangenes Steuersignal anzutreiben, wenn der Schalter in seinem Modus "Ein" ist.

62. Vorrichtung nach Anspruch 53, ferner aufweisend einen äußeren Energiesender zum Übertragen von Funkenergie, wobei die Energieversorgungseinrichtung eine Batterie und einen Schalter aufweist, der durch die von dem äußeren Sender übertragene Funkenergie betätigbar ist, zum Verbinden der Batterie mit dem Signalempfänger in einem Modus "Ein", wenn der Schalter durch die Funkenergie eingeschaltet ist, und um die Batterie von dem Signalempfänger in einem Modus "Bereitschaft" getrennt zu halten, wenn der Schalter ausgeschaltet ist.

63. Vorrichtung nach Anspruch 1, ferner aufweisend eine implantierbare Energieversorgungseinrichtung (336) zum Bereitstellen von Energie an Energieverbrauchskomponenten der in dem Patienten zu implantierenden Vorrichtung.

64. Vorrichtung nach Anspruch 63, ferner aufweisend einen äußeren Energiesender zum Übertragen von Funkenergie, wobei die Energieversorgungseinrichtung geeignet ist, die Funkenergie in elektrische Energie umzuwandeln.

65. Vorrichtung nach Anspruch 64, ferner aufweisend einen implantierbaren Elektromotor (22) zum Betätigen der Einstellvorrichtung (12;52;66;90;92;104;110), wobei die Energieversorgungseinrichtung (326) geeignet ist, den Elektromotor (22) mit der aus der Funkenergie umgewandelten elektrischen Energie anzutreiben.

66. Vorrichtung nach Anspruch 63, ferner aufweisend einen äußeren Energiesender zum Übertragen von Funkenergie, wobei die Energieversorgungseinrichtung eine Batterie und einen Schalter aufweist, der durch die von dem äußeren Sender übertragene Funkenergie betätigbar ist, zum Verbinden der Batterie mit den implantierbaren Energieverbrauchskomponenten der Vorrichtung in einem Modus "Ein", wenn der Schalter durch die Funkenergie eingeschaltet ist, und um die Batterie von den Energieverbrauchskomponenten in einem Modus "Bereitschaft" getrennt zu halten, wenn der Schalter ausgeschaltet ist.

67. Vorrichtung nach Anspruch 62 oder 65, wobei der äußere Energiesender geeignet ist, den Schalter direkt mit der Funkenergie zu aktivieren, um in den Modus "Ein" zu schalten.

68. Vorrichtung nach Anspruch 50, wobei die Fernsteuerung (22;326;332-344) geeignet ist, Informationen von implantierbaren Komponenten der Vorrichtung zu erhalten und der Einstellvorrichtung (12;52,66;90;104;110) zu befehlen, die Begrenzungsvorrichtung (2;48;60;62;88;110;122;126,128; 130;434) in Reaktion auf erhaltene Informationen einzustellen.

69. Vorrichtung nach Anspruch 1, ferner aufweisend implantierbare elektrische Komponenten mit wenigstens einer Spannungspegelschutzvorrichtung.

70. Vorrichtung nach Anspruch 1, ferner aufweisend implantierbare elektrische Komponenten mit einer einzigen Spannungspegelschutzvorrichtung.

71. Vorrichtung nach Anspruch 69 oder 70, wobei die elektrischen Komponenten frei von irgendeinem Stromdetektor und/oder Ladezustandsdetektor sind.

72. Vorrichtung nach einem der Ansprüche 69-71, ferner aufweisend einen implantierbaren Kondensator oder Akkumulator, wobei die Ladung oder Entladung des Kondensators oder Akkumulators durch Verwendung der Spannungspegelschutzvorrichtung gesteuert wird.

73. Vorrichtung nach Anspruch 1, ferner aufweisend ein Energieübertragungsmittel (22,326,332-344) zum drahtlosen Übertragen von Energie von außerhalb des Körpers des Patienten an die Einstellvorrichtung und/oder andere implantierbare Energieverbrauchskomponenten der Vorrichtung.

74. Vorrichtung nach Anspruch 73, wobei das Energieübertragungsmittel geeignet ist, die Energie in der Form einer Reihe von Energieimpulsen zur direkten Verwendung in Verbindung mit der Energieversorgung der Energieverbrauchskomponenten der Vorrichtung intermittierend zu übertragen.

75. Vorrichtung nach Anspruch 74, wobei das Energieübertragungsmittel geeignet ist, elektrische Energie zu übertragen, und ferner aufweisend einen implantierbaren Kondensator zum Erzeugen der Reihe von Energieimpulsen.

76. Vorrichtung nach Anspruch 72 oder 75, wobei der Kondensator eine Kapazität von weniger als 0,1 µF hat.

77. Vorrichtung nach Anspruch 73, ferner aufweisend eine(n) implantierbare(n) Motor (22) oder Pumpe zum Betätigen der Einstellvorrichtung (12;52;66;90,92;104;110), wobei das Energieübertragungsmittel geeignet ist, den Motor oder die Pumpe direkt mit übertragener Energie anzutreiben.

78. Vorrichtung nach Anspruch 1, wobei die angetriebene Betätigungsvorrichtung eine(n) implantierbare(n) Motor oder Pumpe zum Betätigen der Einstellvorrichtung aufweist, wobei die Energieübertragungsvorrichtung geeignet ist, Funkenergie in der Form eines magnetischen Feldes oder elektromagnetischer Wellen zum direkten Antrieb des Motors oder der Pumpe zu übertragen, wenn die Funkenergie übertragen wird.

79. Vorrichtung nach Anspruch 78, wobei die Pumpe keine Pumpe des Plungertyps ist.

80. Vorrichtung nach Anspruch 73, wobei das Energieübertragungsmittel geeignet ist, Funkenergie in der Form von elektromagnetischen Wellen mit Ausnahme von Radiowellen zu übertragen.

81. Vorrichtung nach den Ansprüchen 73 oder 74, wobei die von dem Energieübertragungsmittel übertragene Energie ein elektrisches Feld oder ein magnetisches Feld aufweist.

82. Vorrichtung nach den Ansprüchen 73 oder 74, wobei die von dem Energieübertragungsmittel übertragene Energie ein Signal aufweist.

83. Vorrichtung nach Anspruch 51 oder 82, wobei das Signal analoge oder digitale Impulse aufweist.

84. Vorrichtung nach Anspruch 83, wobei die analogen oder digitalen Impulse ein magnetisches Feld oder ein elektrisches Feld aufweisen.

85. Vorrichtung nach Anspruch 51, 81 oder82, wobei das Signal ein Wellensignal aufweist.

86. Vorrichtung nach Anspruch 85, wobei das Wellensignal ein elektromagnetisches Wellensignal, ein Schallwellensignal oder ein Trägerwellensignal aufweist.

87. Vorrichtung nach einem der vorhergehenden Ansprüche, ferner aufweisend einen Drucksensor zum direkten oder indirekten Erfassen des Druckes gegen die Begrenzungsvorrichtung.

88. Vorrichtung nach Anspruch 87, wobei die Begrenzungsvorrichtung in Reaktion auf Signale von dem Drucksensor gesteuert wird.

89. Vorrichtung nach Anspruch 1, ferner aufweisend eine implantierbare Energieumwandlungsvorrichtung zum direkten oder indirekten Umwandeln von Funkenergie in andere Energie als die Funkenergie für die Betätigung der Begrenzungsvorrichtung.

90. Vorrichtung nach Anspruch 89, wobei die Energieumwandlungsvorrichtung die Funkenergie in kinetische Energie für die Betätigung der Begrenzungsvorrichtung umwandelt.

91. Vorrichtung nach Anspruch 89, wobei die Energieumwandlungsvorrichtung die Funkenergie in der Form von Schallwellen in elektrische Energie für die Betätigung der Begrenzungsvorrichtung umwandelt.

92. Vorrichtung nach einem der vorhergehenden Ansprüche, ferner aufweisend eine implantierbare Umschaltvorrichtung, wobei die Begrenzungsvorrichtung zum Durchführen einer Umkehrfunktion geeignet ist und die Umschaltvorrichtung die Umkehrfunktion umschaltet.

93. Vorrichtung nach einem der vorhergehenden Ansprüche, ferner aufweisend eine(n) implantierbare(n) Akkumulator oder Batterie und Mittel zur Steuerung des Akkumulators oder der Batterie von außerhalb des Körpers des Patienten, um die Einstellvorrichtung und/oder andere implantierbare Energieverbrauchskomponenten der Vorrichtung mit Energie zu versorgen.

94. Vorrichtung nach Anspruch 1, wobei die Einstellvorrichtung geeignet ist, die Begrenzungsvorrichtung in einer nichtmanuellen Weise einzustellen.

95. Vorrichtung nach einem der vorhergehenden Ansprüche, wobei die Begrenzungsvorrichtung für die Implantation in der Basis des Penis oder dessen Verlängerung des Patienten gestaltet ist.

96. Vorrichtung nach Anspruch 91, wobei die Energieumwandlungsvorrichtung die Funkenergie in der Form von Schallwellen direkt in elektrische Energie umwandelt.

97. Vorrichtung nach Anspruch 73, 91 oder 96, wobei die Energieumwandlungsvorrichtung einen Kondensator aufweist.

98. Vorrichtung nach Anspruch 97, wobei der Kondensator geeignet ist, elektrische Impulse aus der umgewandelten elektrischen Energie zu erzeugen.

99. Vorrichtung nach Anspruch 48, wobei der Umschaltservo einen Motor, vorzugsweise einen Elektromotor aufweist.

100. Vorrichtung nach Anspruch 99, wobei der Motor umschaltbar ist.

101. Vorrichtung nach Anspruch 86, wobei das Trägersignal frequenz-, amplituden- oder frequenz- und amplitudenmoduliert ist.

102. Vorrichtung nach Anspruch 86, wobei das Steuersignal ein Wellensignal aufweist, umfassend eines von einem Schallwellensignal, darunter ein Ultraschallwellensignal, einem elektromagnetischen Wellensignal, darunter ein Infrarotlichtsignal, ein sichtbares Lichtsignal, ein Ultraviolettlichtsignal und ein Laserlichtsignal, einem Mikrowellensignal, einem Radiowellensignal, einem Röntgenstrahlungssignal und einem Gammastrahlungssignal.

103. Vorrichtung nach Anspruch 1, wobei das Hydraulikmittel der Betätigungsvorrichtung eine Fluidleitung aufweist, wobei der Behälter einen Teil der Leitung bildet.

104. Vorrichtung nach Anspruch 103, wobei das Hydraulikmittel einschließlich der Leitung frei von irgendeinem Rückschlagventil sind.

105. Vorrichtung nach Anspruch 73, wobei das Energieübertragungsmittel geeignet ist, magnetische Energie, nichtmagnetische Energie, elektromagnetische Energie, nichtelektromagnetische Energie, kinetische Energie, nichtkinetische Energie, akustische Energie, nichtakustische Energie, thermische Energie oder nichtthermische Energie zu übertragen.

106. Vorrichtung nach Anspruch 4, wobei die Begrenzungsvorrichtung geeignet ist, die Querschnittsfläche eines von dem austretenden Penisvenen des Patienten gebildeten Blutflussdurchgangs zu steuern.

107. Vorrichtung nach Anspruch 106, wobei die Begrenzungsvorrichtung betätigbar ist, um den Blutflussdurchgang zu öffnen und zu schließen.

108. Vorrichtung nach Anspruch 107, wobei die Begrenzungsvorrichtung geeignet ist, die Querschnittsfläche des Blutflussdurchgangs stufenlos zu steuern.

## Revendications

1. Appareil de traitement de l'impuissance sexuelle masculine, comprenant un dispositif de restriction réglable (2 ; 48 ; 60,62 ; 88 ; 110 ; 122 ; 126 ; 130 ; 434 ; 444,446) pouvant s'implanter chez un patient impuissant pour mettre en prise directement une partie du tissu pénien normal ou le prolongement de celui-ci, du patient, un dispositif de réglage activable (12 ; 52 ; 66 ; 90,92 ; 104 ; 110 ; 436) pouvant s'implanter chez le patient et conçu pour régler mécaniquement le dispositif de restriction pour contracter temporairement ladite partie du tissu pénien normal ou le prolongement de celui-ci afin de restreindre le flux sanguin quittant le pénis, lorsque le patient désire avoir une érection, et un dispositif d'actionnement implantable pour actionner le dispositif de réglage, dans lequel le dispositif d'actionnement comprend des moyens hydrauliques (54) pour actionner le dispositif de réglage (52) et dans lequel l'appareil comprend en outre un réservoir implantable (204 ; 210 ; 216) contenant une quantité prédéterminée de fluide pour fournir du fluide aux moyens hydrauliques, **caractérisé en ce que** le dispositif d'actionnement est motorisé pour faire fonctionner le dispositif de réglage.

2. Appareil selon la revendication 1, dans lequel le dispositif de restriction est utilisable pour contracter ladite partie du tissu pénien du patient ou le prolongement de celui-ci, lorsque le patient désire avoir une érection, de sorte que le flux sanguin artériel du pénis soit au moins sensiblement non restreint, tandis que le flux sanguin veineux du pénis soit sensiblement restreint.

3. Appareil selon la revendication 1 ou 2, dans lequel le dispositif de restriction est conçu pour s'engager à la fois avec le corps caverneux ou les racines du corps caverneux du pénis ou les prolongements de ces derniers en tant qu'une seule unité, ou pour être en prise avec chacun des deux corps caverneux ou racines du corps caverneux ou le prolongement de ces derniers en tant que deux unités séparées.

4. Appareil selon la revendication 1 ou 2, dans lequel ladite partie du tissu pénien comprend une ou plusieurs veines de sortie du pénis.

5. Appareil selon l'une quelconque des revendications 1 à 4, dans lequel le dispositif de réglage (12 ; 52 ; 66 ; 90,92 ; 104; 110 ; 436) est conçu pour régler le dispositif de restriction de façon non magnétique ou non thermique.

6. Appareil selon l'une quelconque des revendications 1 à 5 dans lequel le dispositif de restriction n'est pas gonflable.

7. Appareil selon l'une quelconque des revendications 1 à 6, dans lequel le dispositif de restriction comprend un élément devant être placé d'un côté de ladite partie du tissu pénien, et le dispositif de réglage est conçu pour presser ladite partie du tissu pénien entre l'élément et l'os ou le tissu humain pour diminuer le flux sanguin quittant le pénis.

8. Appareil selon l'une quelconque des revendications 1 à 6 dans lequel le dispositif de restriction comprend au moins un élément de restriction allongé (2 ; 48 ; 60,62 ; 88) et des moyens de formation (10 ; 94 ; 106 ; 108 ; 118 ; 132) pour former l'élément de restriction dans au moins une boucle sensiblement fermée autour de ladite partie du tissu pénien, la boucle définissant une ouverture de restriction, dans lequel le dispositif de réglage est conçu pour régler l'élément de restriction dans la boucle afin de modifier la dimension de l'ouverture de restriction.

9. Appareil selon la revendication 8, dans lequel le dispositif de restriction comprend plusieurs éléments de restriction allongés (2 ; 48 ; 60,62 ; 88) devant être formés dans des boucles au moins sensiblement fermées autour du tissu pénien.

10. Appareil selon la revendication 8 ou 9, dans lequel le dispositif de réglage (12 ; 52 ; 66 ; 90,92) est conçu pour ajuster l'extension longitudinale de l'élément de restriction allongé dans ladite boucle afin de modifier la dimension de l'ouverture de restriction.

11. Appareil selon la revendication 10, dans lequel l'élément de restriction (2 ; 48) comprend une partie principale et deux parties d'extrémité allongées (4,6) et le dispositif de réglage (12 ; 52 ; 66 ; 90,92) est conçu pour établir le déplacement relatif longitudinal entre les parties d'extrémité de l'élément de restriction, de sorte que l'ouverture de restriction (3) soit ajustée.

12. Appareil selon la revendication 1, dans lequel le dispositif de réglage (12 ; 52 ; 66 ; 90,92) comprend un élément de transfert de mouvement (14 ; 40 ; 78,90 ; 80,92) en prise avec au moins les parties d'extrémité (4, 6) de l'élément de restriction et utilisable pour déplacer ladite partie d'extrémité par rapport à l'autre partie d'extrémité de l'élément de restriction.

13. Appareil selon la revendication 12, comprenant en outre un moteur (22) qui est fixé par rapport à la partie principale de l'élément de restriction (2) et comporte un arbre d'entraînement rotatif (32) connecté de façon opérationnelle à l'élément de transfert de mouvement (14).

14. Appareil selon la revendication 13, dans lequel le moteur est positionné par rapport à l'élément de restriction allongé de sorte que l'arbre d'entraînement s'étende parallèlement à une membrure dans ladite boucle de l'élément de restriction.

15. Appareil selon la revendication 10, dans lequel l'élément de restriction allongé (48, 50) est élastique dans la direction longitudinale et le dispositif de réglage comprend des moyens de contraction (52) conçus pour contracter de façon longitudinale l'élément de restriction élastique.

16. Appareil selon la revendication 15, dans lequel l'élément de restriction allongé comprend une partie principale sensiblement non élastique (48) et une partie d'extrémité formant un ressort hélicoïdal (50), qui peut être contracté par les moyens de contraction (52).

17. Appareil selon la revendication 16, dans lequel les moyens de contraction comprennent un élément de traction souple allongé (52) relié à la partie principale (48) de l'élément de restriction et s'étendant à travers le ressort hélicoïdal (50) pour contracter le ressort hélicoïdal contre un élément d'arrêt (56), qui est fixe par rapport à la partie principale de l'élément de restriction.

18. Appareil selon la revendication 8, dans lequel l'élément de restriction comprend un ressort hélicoïdal allongé (60) ayant une extrémité libre, et un corps (64) auquel ledit ressort est fixé de façon non rotative à son extrémité opposée, le dispositif de réglage (66) étant conçu pour tourner le ressort hélicoïdal dans une direction afin d'élargir les spires du ressort hélicoïdal de façon à contracter longitudinalement le ressort hélicoïdal allongé et pour tourner le ressort hélicoïdal dans la direction opposée afin de réduire la dimension des spires du ressort hélicoïdal pour étendre longitudinalement le ressort hélicoïdal.

19. Appareil selon la revendication 18, dans lequel l'élément de restriction comprend en outre un autre ressort hélicoïdal allongé (62) ayant une extrémité libre et fixé de façon non rotative au corps (64) à son extrémité opposée, et le dispositif de réglage comprend un arbre d'entraînement (66) ayant deux parties d'extrémité opposées reliées aux ressorts hélicoïdaux, respectivement, à leurs extrémités libres, les spires hélicoïdales formant des hélices droites et gauches, respectivement.

20. Appareil selon la revendication 19, dans lequel l'élément de restriction comprend en outre un autre ressort hélicoïdal allongé (62) ayant une extrémité libre et fixé de façon non rotative au corps (64) à son extrémité opposée, et le dispositif de réglage comprend un engrenage (70) ayant un arbre d'entrée (78) et deux arbres de sortie opposés alignés (74, 76) reliés aux ressorts hélicoïdaux (60,62), respectivement, à leurs extrémités libres, l'arbre d'entré étant connecté aux arbres de sortie, de sorte que les arbres de sortie tournent dans des directions opposées lors de la rotation de l'arbre d'entrée, les spires hélicoïdales formant les mêmes hélices.

21. Appareil selon la revendication 8, dans lequel l'élément de restriction (88 ; 110; 122) forme une surface de confinement périphérique interne radiale dans ladite boucle de l'élément de restriction, et le dispositif de réglage (104 ; 112) est conçu pour régler mécaniquement l'élément de restriction de sorte qu'au moins une partie de la surface de confinement soit déplacée sensiblement radialement dans ladite boucle.

22. Appareil selon la revendication 21, dans lequel le dispositif de réglage comprend un élément allongé sensible à une tension (104) faisant partie de la surface de confinement et apte à se courber en réponse à une tension appliquée à travers l'élément, le rayon de courbure de ladite courbe étant réglable en modifiant le niveau de la tension.

23. Appareil selon la revendication 21, dans lequel l'élément de restriction comprend un élément annulaire élastique (110) formant la surface de confinement, et le dispositif de réglage (112) est conçu pour modifier le diamètre de l'élément annulaire élastique.

24. Appareil selon la revendication 21, dans lequel les moyens de formation comprennent un élément annulaire externe sensiblement rigide (118) et l'élément de restriction comprend un ressort hélicoïdal allongé (60) s'étendant de façon interne le long de l'élément annulaire externe et en contact avec celui-ci, le ressort hélicoïdal faisant partie de la surface de confinement périphérique et ayant une extrémité libre, et un corps auquel le ressort hélicoïdal est fixé de façon non rotative à son extrémité opposée, le dispositif de réglage étant conçu pour tourner le ressort hélicoïdal dans une direction afin d'élargir les spires du ressort hélicoïdal de façon à contracter la surface de confinement périphérique et pour tourner le ressort hélicoïdal dans la direction opposée pour réduire la dimension des spires du ressort hélicoïdal afin d'accroître la surface de confinement périphérique.

25. Appareil selon la revendication 21, dans lequel les moyens de formation comprennent un élément annulaire externe sensiblement rigide (118) et l'élément de restriction comprend un premier (60) et un second (62) ressort hélicoïdal allongé s'étendant de façon interne le long de l'élément annulaire externe et en contact avec celui-ci, les ressorts hélicoïdaux faisant partie de la surface de confinement périphérique, le premier et second ressort, respectivement ayant une extrémité libre, et un corps auquel le premier et le second ressort, respectivement est fixé de façon non rotative à son extrémité opposée, le dispositif de réglage étant conçu pour tourner le premier et le second ressort, respectivement dans une direction afin d'élargir les spires du ressort de façon à contracter la surface de confinement périphérique et pour tourner le premier et le second ressort, respectivement dans la direction opposée pour réduire la dimension des spires du ressort afin d'accroître la surface de confinement périphérique.

26. Appareil selon la revendication 8, dans lequel l'élément de restriction comprend au moins deux éléments séparés, l'un d'eux au moins est pivoté de sorte qu'il peut tourner dans un plan dans lequel ladite boucle de l'élément de restriction s'étend, et le dispositif de réglage est conçu pour tourner ledit élément pivoté pour modifier la dimension de ladite ouverture de restriction.

27. Appareil selon l'une quelconque des revendications 1 à 6 dans lequel le dispositif de restriction comprend au moins deux éléments de cadre (126 et 128), qui sont pliables l'un par rapport à l'autre par le dispositif de réglage.

28. Appareil selon la revendication 27, dans lequel les éléments de cadre pliables comprennent deux éléments de cadre sensiblement semi-circulaires (126 et 128), articulés l'un à l'autre de sorte que les éléments semi-circulaires peuvent osciller l'un par rapport à l'autre à partir d'un état entièrement ouvert dans lequel ils forment sensiblement à un état entièrement plié dans lequel ils forment un demi-cercle.

29. Appareil selon la revendication 8, dans lequel l'élément de restriction allongé (130) est élastique et à épaisseur variable comme illustré dans une section transversale de celui-ci, et le dispositif de réglage est conçu pour tourner l'élément de restriction autour de l'extension longitudinale de celui-ci.

30. Appareil selon la revendication 8, dans lequel les moyens de formation comprennent un matériau élastique formant l'élément de restriction allongé dans la boucle, de sorte que l'ouverture de restriction ait une dimension prédéfinie, et le dispositif de réglage soit conçu pour régler l'élément de restriction par rapport à l'action du ressort du matériau élastique.

31. Appareil selon la revendication 30, dans lequel le matériau élastique est intégré dans l'élément de restriction.

32. Appareil selon la revendication 8, dans lequel les moyens de formation (10 ; 94 ; 106 ; 118; 132) sont conçus pour former l'élément de restriction (2; 48 ; 60,62 ; 88 ; 110 ; 122 ; 126 ; 128 ; 130) en une boucle ayant une dimension prédéterminée ou une dimension choisie parmi plusieurs dimensions prédéfinies.

33. Appareil selon la revendication 8, dans lequel le dispositif de réglage (12 ; 52 ; 66 ; 90 ; 104 ; 110) est conçu pour modifier la dimension de l'ouverture de restriction (3) de sorte que la surface de confinement périphérique externe de l'élément de restriction (2 ; 48; 60,62 ; 88 ; 110 ; 122 ; 126 ; 128 ; 130) soit modifiée.

34. Appareil selon la revendication 8, dans lequel le dispositif de réglage (12 ; 52 ; 66 ; 90 ; 104 ; 110 ; 434) est conçu pour modifier la dimension de l'ouverture de restriction (3) de sorte que la surface de confinement périphérique externe de l'élément de restriction (2 ; 48; 60,62 ; 88 ; 110 ; 122 ; 126 ; 128 ; 130 ; 436) ne soit pas modifiée.

35. Appareil selon la revendication 8, dans lequel l'élément de restriction allongé (404) est souple, et le dispositif de réglage (410) est conçu pour tirer une première partie (404A) de l'élément de restriction souple à partir d'une seconde partie (404B) de l'élément de restriction souple opposé à la première partie dans la boucle pour presser ladite partie du tissu pénien (406) entre deux longueurs opposées de l'élément de restriction souple allongé pour restreindre le flux sanguin quittant le pénis, et pour libérer ladite partie du tissu pénien (406) de l'élément de restriction souple afin d'augmenter le flux sanguin quittant le pénis.

36. Appareil selon l'une quelconque des revendications 1 à 6, dans lequel le dispositif de restriction (412) comprend au moins deux éléments (414) à placer sur les différents côtés de ladite partie du tissu pénien (406) et le dispositif de réglage est conçu pour presser ladite partie du tissu pénien (406) entre les éléments pour restreindre le flux sanguin quittant le pénis, et pour libérer ladite partie du tissu pénien (406) desdits éléments afin d'augmenter le flux sanguin quittant le pénis.

37. Appareil selon l'une quelconque des revendications 1 à 6 dans lequel le dispositif de restriction (418) comprend au moins deux éléments de serrage articulés (420) devant être positionnés sur les côtés opposés ou différents de ladite partie du tissu pénien (406) et le dispositif de réglage (422) est conçu pour tourner les éléments de serrage l'un vers l'autre afin de serrer ladite partie du tissu pénien (406) entre les éléments de serrage pour restreindre le flux sanguin quittant le pénis, et pour séparer les éléments de serrage l'un de l'autre pour libérer ladite partie du tissu pénien (406) des éléments de serrage afin d'augmenter le flux sanguin quittant le pénis.

38. Appareil selon l'une quelconque des revendications 1 à 6 dans lequel le dispositif de restriction est conçu pour fléchir une partie de ladite partie du tissu pénien (406).

39. Appareil selon la revendication 38, dans lequel le dispositif de restriction (424) comprend au moins deux éléments de flexion (426-430) devant être positionnés sur des côtés opposés ou différents de ladite partie du tissu pénien (406) et pour être déplacés l'un par rapport à l'autre le long du tissu pénien, et le dispositif de réglage (432) est conçu pour déplacer les éléments de flexion contre ladite partie du tissu pénien (406) pour la fléchir et restreindre le flux sanguin quittant le pénis, et pour déplacer les éléments de flexion à distance de ladite partie de tissu pénien (406) pour la libérer des éléments de flexion afin d'augmenter le flux sanguin quittant le pénis.

40. Appareil selon la revendication 39, dans lequel les éléments de flexion comprennent des rouleaux.

41. Appareil selon l'une quelconque des revendications 1 à 6 dans lequel le dispositif de restriction est conçu pour tourner une partie du tissu pénien.

42. Appareil selon la revendication 1, dans lequel le dispositif d'actionnement comprend un servomécanisme.

43. Appareil selon la revendication 1, dans lequel le réservoir (210) définit une chambre pour la quantité prédéterminée de fluide et le moyen hydraulique (202) du dispositif d'actionnement est conçu pour modifier le volume de la chambre.

44. Appareil selon la revendication 43, dans lequel le moyen hydraulique comprend une pompe (212) conçue pour pomper le liquide entre le réservoir (204) et le dispositif de réglage (202).

45. Appareil selon la revendication 43 ou 44, dans lequel le moyen hydraulique est dépourvu de clapet anti-retour.

46. Appareil selon l'une quelconque des revendications 43 à 45, dans lequel le moyen hydraulique comprend un servomécanisme d'inversion (210 ; 214 ; 276-286).

47. Appareil selon la revendication 46, dans lequel le servomécanisme d'inversion est actionné manuellement.

48. Appareil selon la revendication 46, dans lequel le servomécanisme d'inversion est motorisé.

49. Appareil selon l'une quelconque des revendications 46 à 48, dans lequel le moyen hydraulique comprend des première et seconde parties latérales du réservoir (204), et le servomécanisme d'inversion est conçu pour fournir le déplacement relatif entre les première et seconde parties latérales du réservoir.

50. Appareil selon l'une quelconque des revendications précédentes, comprenant en outre une commande à distance sans fil (22, 326, 332-344) pour commander de façon non invasive le dispositif de réglage.

51. Appareil selon la revendication 50, dans lequel la commande à distance (22,326,332-344) comprend un émetteur et/ou récepteur de signal séparé (332,336) et un récepteur et/ou émetteur de signal implantable (334, 338), pour transmettre et/ou recevoir un signal de commande.

52. Appareil selon la revendication 51, dans lequel le récepteur de signal (334, 338) comprend une unité de commande (338) conçue pour commander le dispositif de réglage (12 ; 52 ; 66 ; 90,92 ; 104 ; 110) en réponse au signal de commande.

53. Appareil selon la revendication 52, comprenant en outre une unité d'alimentation implantable (336) pour fournir de l'énergie à des composants consommant de l'énergie de l'appareil devant être implanté chez le patient.

54. Appareil selon l'une quelconque des revendications 50 à 53, comprenant en outre un moteur implantable (22) pour actionner le dispositif de réglage (12 ; 52 ; 66 ; 90,92; 104 ; 110).

55. Appareil selon les revendications 53 et 54, dans lequel l'unité de commande (338) est conçue pour commander l'unité d'alimentation (336) afin d'alimenter le moteur (22) en énergie en réponse au signal de commande.

56. Appareil selon la revendication 54 ou 55, dans lequel le moteur (22) est un moteur électrique.

57. Appareil selon la revendication 53, dans lequel l'unité d'alimentation (326) est conçue pour transformer l'énergie du signal de commande, lors de la transmission au récepteur de signal (334,338), en énergie électrique.

58. Appareil selon la revendication 53 ou 57, dans lequel l'unité d'alimentation (26) est conçue pour transformer l'énergie du signal de commande en un courant continu ou alternatif.

59. Appareil selon la revendication 58, comprenant en outre un moteur électrique implantable (22) pour actionner le dispositif de réglage (12 ; 52 ; 66 ; 90,92; 104 ; 110) dans lequel l'unité d'alimentation (326) comprend une alimentation électrique rechargeable (58) pour stocker l'énergie électrique et l'unité de commande (338) est conçue pour alimenter le moteur électrique (22) en énergie à partir de l'alimentation électrique rechargeable en réponse au signal de commande.

60. Appareil selon la revendication 53, dans lequel l'unité d'alimentation (326) comprend une batterie, un commutateur actionnable électriquement pour connecter la batterie au récepteur de signal (334, 338) dans un mode « on » lorsque le commutateur est sous tension et pour maintenir la batterie déconnectée du récepteur de signal dans un mode « standby » lorsque le commutateur n'est pas sous tension, et une alimentation électrique rechargeable pour alimenter le commutateur.

61. Appareil selon la revendication 60, dans lequel l'unité de commande (338) est conçue pour alimenter le moteur électrique (22) en énergie depuis la batterie en réponse à un signal de commande reçu de l'émetteur de signal (332, 336), lorsque le commutateur est dans le mode « on ».

62. Appareil selon la revendication 53, comprenant en outre un émetteur d'énergie externe pour transmettre l'énergie sans fil, dans lequel l'unité d'alimentation comprend une batterie et un commutateur actionnable par l'énergie sans fil transmise par l'émetteur externe, pour connecter la batterie au récepteur de signal dans un mode « on » lorsque le commutateur est alimenté par l'énergie sans fil et pour maintenir la batterie déconnectée du récepteur de signal dans un mode « standby » lorsque le commutateur n'est pas alimenté.

63. Appareil selon la revendication 1, comprenant en outre une unité d'alimentation implantable (336) pour fournir de l'énergie à des composants consommant de l'énergie de l'appareil à implanter chez un patient.

64. Appareil selon la revendication 63, comprenant en outre un émetteur d'énergie externe pour transmettre l'énergie sans fil, dans lequel l'unité d'alimentation est conçue pour transformer l'énergie sans fil en énergie électrique.

65. Appareil selon la revendication 64, comprenant en outre un moteur électrique implantable (22) pour actionner le dispositif de réglage (12 ; 52 ; 66 ; 90,92; 104 ; 110), dans lequel l'unité d'alimentation (326) est conçue pour alimenter le moteur électrique (22) avec l'énergie électrique transformée à partir de l'énergie sans fil.

66. Appareil selon la revendication 63, comprenant en outre un émetteur d'énergie externe pour transmettre l'énergie sans fil, dans lequel l'unité d'alimentation comprend une batterie et un commutateur actionnable par l'énergie sans fil transmise par l'émetteur externe, pour connecter la batterie aux composants consommant de l'énergie implantables de l'appareil dans un mode « on » lorsque le commutateur est alimenté par l'énergie sans fil et pour maintenir la batterie déconnectée des composants consommant de l'énergie dans un mode « standby » lorsque le commutateur n'est pas alimenté.

67. Appareil selon la revendication 62 ou 65, dans lequel l'émetteur d'énergie externe est conçu pour alimenter directement le commutateur avec l'énergie sans fil pour commuter dans le mode « on ».

68. Appareil selon la revendication 50, dans lequel la commande à distance (22, 326, 332-344) est capable d'obtenir des informations des composants implantables de l'appareil et de commander le dispositif de réglage (12 ; 52 ; 66 ; 90 ; 104 ; 110) pour régler le dispositif de restriction (2; 48; 60,62; 88; 110; 122 ; 126 ; 128; 130 ; 434) en réponse aux informations obtenues.

69. Appareil selon la revendication 1, comprenant en outre des composants électriques implantables comprenant au moins un contrôle de niveau de tension.

70. Appareil selon la revendication 1, comprenant en outre des composants électriques implantables comprenant un seul contrôle de niveau de tension.

71. Appareil selon la revendication 69 ou 70, dans lequel les composants électriques sont dépourvus de tout détecteur de courant et/ou détecteur de niveau de charge.

72. Appareil selon l'une quelconque des revendications 69 à 71, comprenant en outre un condensateur ou accumulateur implantable, dans lequel la charge ou la décharge du condensateur ou accumulateur est contrôlée par l'utilisation du contrôle de niveau de tension.

73. Appareil selon la revendication 1, comprenant en outre des moyens de transfert d'énergie (22,326,332-344) pour le transfert d'énergie sans fil depuis l'extérieur du corps du patient vers le dispositif de réglage et/ou autres composants implantables consommant de l'énergie de l'appareil.

74. Appareil selon la revendication 73, dans lequel les moyens de transfert d'énergie sont conçus pour transférer de façon intermittente l'énergie sous la forme d'un train d'impulsion d'énergie pour utilisation directe en connexion avec l'alimentation des composants consommant de l'énergie de l'appareil.

75. Appareil selon la revendication 74, dans lequel les moyens de transfert d'énergie sont conçus pour transférer l'énergie électrique, et comprenant en outre un condensateur implantable pour produire le train d'impulsions d'énergie.

76. Appareil selon la revendication 72 ou 75, dans lequel le condensateur a une capacité inférieure à 0,1 µF.

77. Appareil selon la revendication 73, comprenant en outre un moteur (22) ou une pompe implantable pour actionner le dispositif de réglage (12 ; 52 ; 66 ; 90,92 ; 104 ; 110) dans lequel les moyens de transfert d'énergie sont conçus pour alimenter directement le moteur ou pompe avec l'énergie transférée.

78. Appareil selon la revendication 1, dans lequel le dispositif de fonctionnement motorisé comprend un moteur ou une pompe implantable pour activer le dispositif de réglage, dans lequel le dispositif de transmission d'énergie est conçu pour transmettre l'énergie sans fil sous la forme d'un champ magnétique ou d'une onde électromagnétique pour l'alimentation directe du moteur ou de la pompe, pendant la transmission de l'énergie sans fil.

79. Appareil selon la revendication 78, dans lequel la pompe n'est pas une pompe de type plongeur.

80. Appareil selon la revendication 73, dans lequel les moyens de transfert d'énergie sont conçus pour transférer l'énergie sans fil sous la forme d'ondes électromagnétiques à l'exclusion des ondes radio.

81. Appareil selon les revendications 73 ou 74, dans lequel l'énergie transférée par les moyens de transfert d'énergie comprend un champ électrique ou un champ magnétique.

82. Appareil selon les revendications 73 ou 74, dans lequel l'énergie transférée par les moyens de transfert d'énergie comprend un signal.

83. Appareil selon les revendications 51 ou 82, dans lequel le signal comprend des impulsions numériques ou analogiques.

84. Appareil selon la revendication 83, dans lequel les impulsions numériques ou analogiques comprennent un champ magnétique ou un champ électrique.

85. Appareil selon la revendication 51, 81 ou 82, dans lequel le signal comprend un signal d'onde.

86. Appareil selon la revendication 85, dans lequel le signal d'onde comprend un signal d'onde électromagnétique, un signal d'onde sonore ou un signal d'onde porteur.

87. Appareil selon l'une quelconque des revendications précédentes comprenant en outre un capteur de pression pour détecter directement ou indirectement la pression contre le dispositif de restriction.

88. Appareil selon la revendication 87, dans lequel le dispositif de restriction est commandé en réponse aux signaux du capteur de pression.

89. Appareil selon la revendication 1, comprenant en outre un dispositif de transformation d'énergie implantable pour transformer l'énergie sans fil directement ou indirectement en énergie différente de l'énergie sans fil pour l'actionnement du dispositif de restriction.

90. Appareil selon la revendication 89, dans lequel le dispositif de transformation d'énergie transforme l'énergie sans fil en énergie cinétique pour l'actionnement du dispositif de restriction.

91. Appareil selon la revendication 89, dans lequel le dispositif de transformation d'énergie transforme l'énergie sans fil sous la forme d'ondes sonores en énergie électrique pour l'actionnement du dispositif de restriction.

92. Appareil selon l'une quelconque des revendications précédentes, comprenant en outre un dispositif d'inversion implantable, dans lequel le dispositif de restriction est capable de réaliser une fonction réversible et le dispositif d'inversion inverse la fonction réversible.

93. Appareil selon l'une quelconque des revendications précédentes, comprenant en outre un accumulateur ou une batterie implantable et des moyens pour commander l'accumulateur ou la batterie depuis l'extérieur du corps du patient pour amener l'énergie au dispositif de réglage et/ou à d'autres composants implantables de l'appareil qui consomment de l'énergie.

94. Appareil selon la revendication 1, dans lequel le dispositif de réglage est conçu pour régler le dispositif de restriction de façon non manuelle.

95. Appareil selon l'une quelconque des revendications précédentes, dans lequel le dispositif de restriction est conçu pour être implanté dans la base du pénis du patient ou son prolongement.

96. Appareil selon la revendication 91, dans lequel le dispositif de transformation d'énergie transforme l'énergie sans fil sous la forme d'ondes sonores directement en énergie électrique.

97. Appareil selon la revendication 73, 91 ou 96, dans lequel le dispositif de transformation d'énergie comprend un condensateur.

98. Appareil selon la revendication 97, dans lequel le condensateur est conçu pour produire des impulsions électriques à partir de l'énergie électrique transformée.

99. Appareil selon la revendication 48, dans lequel le servomécanisme d'inversion comprend un moteur, de préférence un moteur électrique.

100. Appareil selon la revendication 99, dans lequel le moteur est réversible.

101. Appareil selon la revendication 86, dans lequel le signal porteur est à modulation de fréquence, d'amplitude ou à modulation de fréquence et d'amplitude.

102. Appareil selon la revendication 86, dans lequel le signal de commande comprend un signal d'onde comprenant un signal d'onde sonore incluant un signal d'onde ultrasonore, un signal d'onde électromagnétique incluant un signal de lumière infrarouge, un signal de lumière visible, un signal de lumière ultraviolette, un signal de lumière laser, un signal micro-onde, un signal d'onde radio, un signal de rayonnement de rayons x, et un signal de rayonnements gamma.

103. Appareil selon la revendication 1, dans lequel le moyen hydraulique du dispositif de fonctionnement comprend un conduit de fluide, le réservoir faisant partie du conduit.

104. Appareil selon la revendication 103, dans lequel le moyen hydraulique comprenant le conduit est dépourvu de clapet anti-retour.

105. Appareil selon la revendication 73, dans lequel les moyens de transfert d'énergie sont conçus pour transférer l'énergie magnétique, l'énergie non magnétique, l'énergie électromagnétique, l'énergie non électromagnétique, l'énergie cinétique, l'énergie non cinétique, l'énergie sonique, l'énergie non sonique, l'énergie thermique, l'énergie non thermique.

106. Appareil selon la revendication 4, dans lequel le dispositif de restriction est conçu pour commander la surface transversale d'une voie de passage du flux sanguin formée par les veines de sortie péniennes du patient.

107. Appareil selon la revendication 106, dans lequel le dispositif de restriction est utilisé pour ouvrir et fermer la voie de passage du flux sanguin.

108. Appareil selon la revendication 107, dans lequel le dispositif de restriction est conçu pour commander en continu la surface transversale de la voie de passage du flux sanguin.
